(19) Europäisches Patentamt
European Patent Office
Office européen des brevets

(11) EP 1 071 671 B1

(12) **EUROPÄISCHE PATENTSCHRIFT**

(45) Veröffentlichungstag und Bekanntmachung des Hinweises auf die Patenterteilung:
**03.08.2005 Patentblatt 2005/31**

(21) Anmeldenummer: **99915760.5**

(22) Anmeldetag: **14.04.1999**

(51) Int Cl.$^7$: **C07D 249/12**, C07D 401/04 // (C07D401/04, 249:00, 213:00)

(86) Internationale Anmeldenummer:
**PCT/EP1999/002498**

(87) Internationale Veröffentlichungsnummer:
**WO 1999/054315 (28.10.1999 Gazette 1999/43)**

(54) **TRIAZOLONE MIT NEUROPROTEKTIVER WIRKUNG**
TRIAZOLONES WITH A NEUROPROTECTIVE ACTION
TRIAZOLONES A EFFET NEUROPROTECTEUR

(84) Benannte Vertragsstaaten:
**AT BE CH CY DE DK ES FI FR GB GR IE IT LI LU MC NL PT SE**

(30) Priorität: **17.04.1998 DE 19816882**

(43) Veröffentlichungstag der Anmeldung:
**31.01.2001 Patentblatt 2001/05**

(73) Patentinhaber: **Boehringer Ingelheim Pharma GmbH & Co.KG**
**55218 Ingelheim am Rhein (DE)**

(72) Erfinder:
- **BRENNER, Michael**
  **D-55411 Bingen am Rhein (DE)**
- **BECHTEL, Wolf-Dietrich**
  **D-55437 Appenheim (DE)**
- **PALLUK, Rainer**
  **D-55411 Bingen am Rhein (DE)**
- **WIENRICH, Marion**
  **D-64331 Weiterstadt (DE)**
- **WEISER, Thomas**
  **D-55268 Nieder-Olm (DE)**

(74) Vertreter: **Kläs, Heinz-Gerd, Dr. et al**
**Boehringer Ingelheim GmbH**
**Binger Strasse 173**
**55216 Ingelheim/Rhein (DE)**

(56) Entgegenhaltungen:

| | |
|---|---|
| **EP-A- 0 185 401** | **EP-A- 0 270 061** |
| **EP-A- 0 273 309** | **EP-A- 0 273 310** |
| **WO-A-93/23382** | **WO-A-97/03067** |
| **US-A- 5 436 252** | **US-A- 5 756 755** |

- **BUSCEMI, SILVESTRE ET AL: "Photoinduced Molecular Rearrangements. The Photochemistry of Some 1,2,4-Oxadiazoles in the Presence of Nitrogen Nucleophiles. Formation of 1,2,4-Triazoles, Indazoles, and Benzimidazoles" J. ORG. CHEM. (1996), 61(24), 8397-8401 , XP002120606**
- **KANE, JOHN M. ET AL: "2,4-Dihydro-3H-1,2,4-triazol-3-ones as anticonvulsant agents" J. MED. CHEM. (1990), 33(10), 2772-7 , XP002120607**
- **RAE, A. DAVID ET AL: "Heterocyclic tautomerism. IV. The solution and crystal structures of 1-aryl-3-phenyl-1,2,4-triazol-5-ones" AUST. J. CHEM. (1988), 41(4), 419-28 , XP002120608**
- **GANDOLFI, R. ET AL: "Site specificity in cycloadditions of diphenylnitrile imine with 8-substituted 8-azaheptafulvenes and tricarbonyl (8-azaheptafulene) iron complexes" TETRAHEDRON (1980), 36(7), 935-41 , XP002120609**
- **BERNARDINI, ANTOINE ET AL: "Electron impact fragmentation of 4-amino-1,2,4-triazol-5-ones" ORG. MASS SPECTROM. , Bd. 14, Nr. 7, 1979, Seiten 369-378, XP002120610**
- **GEORGE, BABU ET AL: "Heterocycles from N-ethoxycarbonylthioamides and dinucleophilic reagents. 1. Dihydro-1,2,4-triazolones and 1,2,4-oxadiazolones" J. ORG. CHEM. (1976), 41(20), 3233-7 , XP002120611**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**(Forts. nächste Seite)**

- **KAMETANI, TETSUJI ET AL: "Syntheses of heterocyclic compounds. CCCLXVII. Syntheses of azole derivatives. III. Syntheses of 1-phenyl-.DELTA.2-1,2,4-triazolin-5- one and 4-phenyl-.DELTA.2-1,3,4-oxadiazolin-5-one derivatives by fusion of 1-phenyl-2-acylhydrazine with urea" J. HETEROCYCL. CHEM. (1970), 7(4), 821-9 , XP002120612**
- **TSUGE, O. ET AL: "Studies of thiobenzoyl isocyanate. I. Reaction of thiobenzoyl isocyanate with hydrazines" TETRAHEDRON (1968), 24(14), 5205-14 , XP002120613**
- **TEBIB S ET AL: "The active analog approach applied to the pharmacophore identification of benzodiazepine receptor ligands" JOURNAL OF COMPUTER-AIDED MOLECULAR DESIGN, Bd. 1, Nr. 2, 1987, Seiten 153-170, XP002106517 ISSN: 0920-654X**


Bemerkungen:

Die Akte enthält technische Angaben, die nach dem Eingang der Anmeldung eingereicht wurden und die nicht in dieser Patentschrift enthalten sind.

## Beschreibung

Die Erfindung betrifft Triazolone zur Verwendung als Arzneimittel, insbesondere als Arzneimittel mit neuroprotektiver Wirkung

Triazolone sind aus dem Stand der Technik bekannt und werden beispielsweise durch die Deutschen Offenlegungsschriften DE 19521162 und DE 3631511 sowie durch die Europäischen Patentanmeldungen EP 270 061 und EP 208 321 offenbart. Die dort offenbarten Verbindungen weisen eine Wirksamkeit als Schädlingsbekämpfungsmittel auf und können insbesondere als Insektizide und Akarizide Verwendung finden.

Die vorliegende Erfindung offenbart im Gegensatz dazu Triazolone, die als Arzneimittel, insbesondere als Arzneimittel mit neuroprotektiver Wirkung, eingesetzt werden können. Überraschenderweise wurde gefunden, daß die erfindungsgemäßen Verbindungen eine Affinität zu oder Aktivität an verschiedenen Rezeptortypen zeigen und eine neuroprotektive Wirkung aufweisen.

*In vitro* und *in vivo* Versuche haben gezeigt, daß die im Gehirn infolge von Hypoglykämie, Hypoxie, Anoxie, globale und fokale Ischämie, Schädel-Hirn-Trauma, Hirn-Ödem und Hirn-Druck auftretenden Zellschäden und Funktionsausfälle z. T. auf einer erhöhten synaptischen Aktivität und somit vermehrter Transmitterfreisetzung beruhen. Neben Glutamat sind Histamin und Serotonin als Neurotransmitter von besonderer Bedeutung. Darüberhinaus werden die Konzentrationen von insbesondere Calcium und Natrium Ionen verändert.

Es ist bekannt, daß nach systemischer Applikation von Glutamat Neuronen im Gehirn von Mäusen zerstört werden (S.M. Rothman und T.W. Olney, Trends in Neurosciences 10 (1987) 299). Dieser Befund läßt unter anderem den Schluß zu, daß Glutamat eine Rolle bei neurodegenerativen Erkrankungen spielt (R.Schwarcz und B. Meldrum, The Lancet 11 (1985) 140). Weiterhin sind Substanzen wie z.B. Quisqualinsäure, Kaininsäure, Ibotensäure, Glutaminsäure, N-Methyl-D-asparaginsäure (NMDA) und $\alpha$-Amino-3-hydroxy-5-methyl-4-isooxazol-propionsäure (AMPA) als exogene bzw. endogene Neurotoxine bekannt. Gehirnläsionen, die mit solchen Substanzen induziert werden können, sind vergleichbar mit jenen, welche mit Zusammenhang mit Epilepsie und anderen neurodegenerativen Erkrankungen - wie z.B. Morbus Huntington und Morbus Alzheimer - auftreten. Substanzen und Ionen, welche die Aktivität der Glutamat-Rezeptoren und des mit diesem Rezeptor verbundenen Ionenkanals hemmen - wie z.B. kompetitive und nichtkompetitive Antagonisten exzitatorischer Aminosäuren - schützen Gehirnzellen vor hypoxischen bzw. ischämischen Schäden. Diese Befunde zeigen, daß die Glutamat-Rezeptoren eine wichtige Rolle bei der Vermittlung des ischämischen Schadens spielen.

Es wurde überraschenderweise gefunden, daß die erfindungsgemäßen Triazolone eine antagonistische Aktivität am AMPA-Rezeptor aufweisen. Darüber hinaus zeigen diese Verbindungen eine hohe Affinität zu folgendem Rezeptortyp: "$Na^+$ Kanal site 2" Bindungsstelle. Aufgrund dieser Befunde können die erfindungsgemäßen Verbindungen bei neurodegenerativen Erkrankungen sowie Gehirnischämie verschiedener Genese eingesetzt werden.

Die Erfindung betrifft Verbindungen der allgemeinen Formel (I) zur Verwendung als Arzneimittel, insbesondere als Arzneimittel mit neuroprotektiver Wirkung,

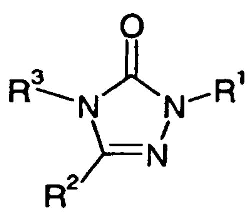

(I)

worin

$R^1$ $C_6$-$C_{10}$-Aryl, bevorzugt Phenyl, das gegebenenfalls direkt oder über eine Alkylenbrücke mit 1 bis 4 C-Atomen durch einen oder mehrere der Reste Halogen, Nitro, $-CF_3$, -CN, $-OR^4$, $-COOR^4$, $-OCOR^4$, $-SR^5$, $-SO_2R^5$, $-OSO_2R^5$, $-NR^6R^7$, $C_1$-$C_4$-Alkyl, $C_2$-$C_4$-Alkenyl oder $C_2$-$C_4$-Alkinyl substituiert sein kann;

$R^2$ $C_6$-$C_{10}$-Aryl, bevorzugt Phenyl, das gegebenenfalls direkt oder über eine Alkylenbrücke mit 1 bis 4 C-Atomen durch einen oder mehrere der Reste Halogen, Nitro, $-CF_3$, -CN, $-OR^4$, $-COOR^4$, $-OCOR^4$, $-SR^5$, $-SO_2R^5$, $-OSO_2R^5$, $-NR^6R^7$, $C_1$-$C_4$-Alkyl, $C_2$-$C_4$-Alkenyl oder $C_2$-$C_4$-Alkinyl substituiert sein kann;

$R^2$ ein $C_1$-$C_6$-Alkyl-, $C_2$-$C_6$-Alkenyl- oder $C_2$-$C_6$-Alkinyl-Rest, der gegebenenfalls durch Phenyl, $-NR^6R^7$, Halogen,

Nitro, $CF_3$, CN oder $-OR^4$ substituiert sein kann;

$R^2$ ein C-verknüpfter 5- oder 6-gliedriger gesättigter oder ungesättigter Heterocyclus, der als Heteroatome 1, 2, 3 oder 4 Atome ausgewählt aus der Gruppe Sauerstoff, Stickstoff oder Schwefel enthalten kann und der gegebenenfalls durch $C_1$-$C_6$-Alkyl oder Benzyl substituiert sein kann;

$R^3$ Wasserstoff oder ein $C_1$-$C_6$-Alkyl-, $C_2$-$C_6$-Alkenyl- oder $C_2$-$C_6$-Alkinyl-Rest, der gegebenenfalls durch $-NR^6R^7$, Halogen, Nitro, $CF_3$, CN oder $-OR^4$ substituiert sein kann;

$R^4$ Wasserstoff, gegebenenfalls durch Halogen oder $-NR^6R^7$ substituiertes $C_1$-$C_4$-Alkyl oder ein Phenyl- oder Benzyl-Rest, der gegebenenfalls eine oder mehrere Methoxygruppen tragen kann;

$R^5$ Wasserstoff, $C_1$-$C_4$-Alkyl, Phenyl oder Benzyl, wobei der Phenyl oder Benzyrest gegebenenfalls ein- oder mehrfach durch Methoxy substituiert sein können;

$R^6$ Wasserstoff, $C_3$-$C_6$-Cycloalkyl, $C_1$-$C_6$-Alkyl, $C_2$-$C_6$-Alkenyl oder $C_2$-$C_6$-Alkinyl, die jeweils ein- oder mehrfach durch Phenyl, Benzyl oder-$OR^4$ substituiert sein können;

$R^6$ $C_6$-$C_{10}$-Aryl, bevorzugt Phenyl, oder Benzyl, die gegebenenfalls durch Halogen, $OR^4$, $C_1$-$C_4$-Alkyl, bevorzugt $-CH_3$, $-SO_3H$, oder $-COOR^4$ substituiert sein können;

$R^7$ Wasserstoff, $C_3$-$C_6$-Cycloalkyl, $C_1$-$C_6$-Alkyl, $C_2$-$C_6$-Alkenyl oder $C_2$-$C_6$-Alkinyl, die jeweils ein- oder mehrfach durch Phenyl, Benzyl oder-$OR^4$ substituiert sein können;

$R^7$ $C_6$-$C_{10}$-Aryl, bevorzugt Phenyl, oder Benzyl, die gegebenenfalls durch Halogen, $OR^4$, $C_1$-$C_4$-Alkyl, bevorzugt $-CH_3$, $-SO_3H$, oder $-COOR^4$ substituiert sein können;
oder
$R^6$ und $R^7$ bilden zusammen mit dem Stickstoffatom einen gesättigten oder ungesättigten 5- oder 6-gliedrigen Ring, der als weitere Heteroatome Stickstoff, Sauerstoff oder Schwefel enthalten kann, wobei der Heterocyclus durch eine verzweigte oder unverzweigte Alkylgruppe mit 1 bis 4 Kohlenstoffatomen, durch Phenyl oder durch Benzyl substituiert sein kann, bedeuten.

Bevorzugt sind Verbindungen der allgemeinen Formel (I) zur Verwendung als Arzneimittel, insbesondere als Arzneimittel mit neuroprotektiver Wirkung, worin

$R^1$ Phenyl, das gegebenenfalls direkt oder über eine Alkylenbrücke mit 1 bis 4 C-Atomen durch einen oder mehrere der Reste Fluor, Chlor, Brom, Nitro, $-CF_3$, -CN, $-OR^4$, $-COOR^4$, $-OCOR^4$, $-NR^6R^7$, $C_1$-$C_4$-Alkyl, $C_2$-$C_4$-Alkenyl oder $C_2$-$C_4$-Alkinyl substituiert sein kann;

$R^2$ Phenyl, das gegebenenfalls direkt oder über eine Alkylenbrücke mit 1 bis 4 C-Atomen durch einen oder mehrere der Reste Fluor, Chlor, Brom, Nitro, $-CF_3$, -CN, $-OR^4$, $-COOR^4$, $-OCOR^4$, $-NR^6R^7$, $C_1$-$C_4$-Alkyl, $C_2$-$C_4$-Alkenyl oder $C_2$-$C_4$-Alkinyl substituiert sein kann;

$R^2$ $C_1$-$C_4$-Alkyl, das gegebenenfalls durch Phenyl substituiert sein kann;

$R^2$ ein C-verknüpfter 5- oder 6-gliedriger gesättigter oder ungesättigter Heterocyclus, der als Heteroatome 1, 2, 3 oder 4 Atome ausgewählt aus der Gruppe Sauerstoff oder Stickstoff enthalten kann und der gegebenenfalls durch $C_1$-$C_4$-Alkyl oder Benzyl substituiert sein kann;

$R^3$ Wasserstoff oder ein $C_1$-$C_4$-Alkyl-Rest, der gegebenenfalls durch $-NR^6R^7$, Fluor, Chlor, Brom, Nitro, $CF_3$, CN oder $-OR^4$ substituiert sein kann;

$R^4$ Wasserstoff, gegebenenfalls durch Halogen oder $-NR^6R^7$ substituiertes $C_1$-$C_4$-Alkyl oder ein Phenyl- oder Benzyl-Rest, der gegebenenfalls eine oder mehrere Methoxygruppen tragen kann;

$R^6$ Wasserstoff, $C_1$-$C_4$-Alkyl, $C_2$-$C_4$-Alkenyl oder $C_2$-$C_4$-Alkinyl, die jeweils ein- oder mehrfach durch Phenyl, Benzyl oder $-OR^4$ substituiert sein können,

$R^6$ Phenyl oder Benzyl, die gegebenenfalls durch Halogen, $OR^4$, $C_1$-$C_4$-Alkyl, bevorzugt $-CH_3$, $-SO_3H$, oder $-COOR^4$ substituiert sein können;

$R^7$ Wasserstoff, $C_1$-$C_4$-Alkyl, $C_2$-$C_4$-Alkenyl oder $C_2$-$C_4$-Alkinyl, die jeweils ein- oder mehrfach durch Phenyl, Benzyl oder $-OR^4$ substituiert sein können,

$R^7$ Phenyl oder Benzyl, die gegebenenfalls durch Halogen, $OR^4$, $C_1$-$C_4$-Alkyl, bevorzugt $-CH_3$, $-SO_3H$, oder $-COOR^4$ substituiert sein können;
oder
$R^6$ und $R^7$ bilden zusammen mit dem Stickstoffatom einen gesättigten oder ungesättigten 5- oder 6-gliedrigen Ring, der als weitere Heteroatome Stickstoff oder Sauerstoff enthalten kann, wobei der Heterocyclus durch eine verzweigte oder unverzweigte Alkylgruppe mit 1 bis 4 Kohlenstoffatomen, durch Phenyl oder durch Benzyl substituiert sein kann, bedeuten.

Besonders bevorzugt sind Verbindungen der allgemeinen Formel (I) zur Verwendung als Arzneimittel, insbesondere als Arzneimittel mit neuroprotektiver Wirkung, worin

$R^1$ Phenyl, das gegebenenfalls durch einen oder mehrere der Reste Fluor, Chlor, Brom, $-OR^4$ oder $C_1$-$C_4$-Alkyl substituiert sein kann;

$R^2$ $C_1$-$C_4$-Alkyl oder Phenyl, das gegebenenfalls durch einen oder mehrere der Reste Fluor, Chlor, Brom, $-OR^4$ oder $C_1$-$C_4$-Alkyl substituiert sein kann;

$R^2$ ein C-verknüpfter 5- oder 6-gliedriger Heterocyclus ausgewählt aus der Gruppe Furan, Pyran, Pyrrol, Pyrazol, Imidazol, Triazol,Tetrazol, Pyridin, Pyridazin, Pyrimidin, Pyrazin, Triazin, Oxazol oder Isoxazol;

$R^3$ Wasserstoff oder ein $C_1$-$C_4$-Alkyl-Rest, der gegebenenfalls durch $-NR^6R^7$, Chlor, Brom oder -OH substituiert sein kann;

$R^4$ Wasserstoff, gegebenenfalls durch Chlor, Brom oder $-NR^6R^7$ substituiertes $C_1$-$C_4$-Alkyl oder ein Phenyl- oder Benzyl-Rest;

$R^6$ Wasserstoff, $C_1$-$C_4$-Alkyl, Phenyl oder Benzyl;

$R^7$ Wasserstoff, $C_1$-$C_4$-Alkyl, Phenyl oder Benzyl; oder
$R^6$ und $R^7$ bilden zusammen mit dem Stickstoffatom einen gesättigten oder ungesättigten 5- oder 6-gliedrigen und gegebenenfalls durch $C_1$-$C_4$-Alkyl oder Benzyl substituierten Ring, ausgewählt aus der Gruppe Piperidin, Piperazin, Morpholin, Pyrrol oder Pyrrolidin, bedeuten.

Besonders bevorzugt sind ferner Verbindungen der allgemeinen Formel (I) zur Verwendung als Arzneimittel, insbesondere als Arzneimittel mit neuroprotektiver Wirkung, worin

$R^1$ Phenyl, das gegebenenfalls durch einen oder mehrere der Reste Fluor, Chlor, Brom, Methyl, Ethyl, Propyl oder $-OR^4$ substituiert sein kann;

$R^2$ Methyl, Ethyl, Propyl, Butyl oder Phenyl, das gegebenenfalls durch einen oder mehrere der Reste Fluor, Chlor, Brom, Methyl, Ethyl, Propyl oder $-OR^4$ substituiert sein kann;

$R^2$ ein C-verknüpfter Heterocyclus ausgewählt aus der Pyrrol, Pyrazol, Imidazol, Triazol, Pyridin, Pyridazin, Pyrimidin, Pyrazin oder Triazin;

$R^3$ Wasserstoff, Methyl, Ethyl oder Propyl die gegebenenfalls durch $-NR^6R^7$, Chlor, Brom oder -OH substituiert sein können;

$R^4$ Wasserstoff, Methyl, Ethyl oder Propyl, die gegebenenfalls durch $-NR^6R^7$, Chlor, Brom oder -OH substituiert sein können;

$R^6$ Wasserstoff, Methyl Ethyl, Propyl oder Benzyl;

$R^7$ Wasserstoff, Methyl Ethyl, Propyl oder Benzyl; oder
$R^6$ und $R^7$ bilden zusammen mit dem Stickstoffatom einen gegebenenfalls durch Methyl Ethyl, Propyl oder Benzyl substituierten Ring, ausgewählt aus der Gruppe Piperidin, Piperazin, Morpholin, Pyrrol oder Pyrrolidin, bedeuten.

Erfindungsgemäß von besonderem Interesse sind Verbindungen der allgemeinen Formel (I) zur Verwendung als Arzneimittel, insbesondere als Arzneimittel mit neuroprotektiver Wirkung, worin

$R^1$ Phenyl, das gegebenenfalls durch einen oder mehrere der Reste Fluor, Chlor, Brom, Methyl, Ethyl, Propyl oder $-OR^4$ substituiert sein kann;

$R^2$ Methyl, Ethyl, Propyl, Butyl oder Phenyl, das gegebenenfalls durch einen oder mehrere der Reste Fluor, Chlor, Brom, Methyl, Ethyl, Propyl oder $-OR^4$ substituiert sein kann;

$R^2$ Pyrrol, Pyrazol, Imidazol, Pyridin, Pyridazin, Pyrimidin oder Pyrazin;

$R^3$ Wasserstoff, Methyl, Ethyl oder Propyl, die gegebenenfalls durch $-NR^6R^7$ sein können;

$R^4$ Methyl, Ethyl, Propyl, $-CH_2CH_2-NR^6R^7$ oder $-CH_2CH_2CH_2-NR^6R^7$;

$R^6$ Wasserstoff, Methyl, Ethyl, Propyl oder Benzyl;

$R^7$ Wasserstoff, Methyl, Ethyl, Propyl oder Benzyl; bedeuten.

Von besonderem Interesse sind ferner die Verbindungen der allgemeinen Formel (I) zur Verwendung als Arzneimittel, insbesondere als Arzneimittel mit neuroprotektiver Wirkung, worin

$R^1$ Benzyl oder Phenyl, das gegebenenfalls durch einen oder mehrere der Reste Fluor, Chlor, Brom, Methyl, Ethyl oder $-OR^4$ substituiert sein kann;

$R^2$ Methyl, Phenyl, das gegebenenfalls durch einen oder mehrere der Reste Chlor, Methyl oder $-OR^4$ substituiert sein kann;

$R^3$ Wasserstoff, Methyl, $-CH_2CH_2-NR^6R^7$ oder $-CH_2CH_2CH_2-NR^6R^7$;

$R^4$ Methyl oder $-CH_2CH_2-NR^6R^7$;

$R^6$ Methyl;

$R^7$ Methyl, bedeuten.

Gegenstand der Erfindung sind ferner pharmazeutische Zusammensetzungen, insbesondere pharmazeutische Zusammensetzungen mit neuroprotektiver Wirkung enthaltend als Wirkstoff einen oder mehrere Verbindungen der allgemeinen Formel (I), worin die Reste $R^1$, $R^2$ und $R^3$ die zuvor genannte Bedeutung aufweisen.

Die Verwendung der Verbindungen der allgemeinen Formel (I) schließt die Verwendung der gegebenenfalls vorliegenden Enantiomere oder Diastereomere in optisch reiner Form oder als Gemische mit ein. Desweiteren können die Verbindungen der allgemeinen Formel (I) in ihre Salze, insbesondere für die pharmazeutische Anwendung in ihre physiologisch verträglichen Salze mit einer anorganischen oder organischen Säure, überführt werden. Als Säuren kommen hierfür beispielsweise Salzsäure, Bromwasserstoffsäure, Schwefelsäure, Phosphorsäure, Essigsäure, Fumarsäure, Bernsteinsäure, Milchsäure, Methansulfonsäure, Zitronensäure, Weinsäure oder Maleinsäure in Betracht. Ferner können Mischungen der vorgenannten Säuren eingesetzt werden.

Der Nachweis der Wirkung der Verbindungen der allgemeinen Formel (I) am AMPA Rezeptor wurde mittels Elektrophysiologie an neuronalen Zellen (Patch-Clamp-Methode) über literaturbekannte Verfahren geführt (M. L. Mayer, L. Vyklicky and G. L. Westbrook, J. Physiol. 415 (1989) 329-350). Der Nachweis der Affinität der Triazolone der allgemeinen Formel (I) zur "$Na^+$ Kanal site 2"-Bindungsstelle wurde wie von G.B. Brown (J. Neurosci. 6 (1986) 2064) beschrieben geführt.

Als Alkylgruppen (auch soweit sie Bestandteil anderer Reste sind, beispielsweise Alkylenbrücken) werden, soweit nicht anders angegeben, verzweigte und unverzweigte Alkylgruppen mit 1 bis 6 Kohlenstoffatomen bevorzugt 1 - 4 Kohlenstoffatomen bezeichnet, beispielsweise werden genannt: Methyl, Ethyl, n-Propyl, iso-Propyl, Butyl, iso-Butyl, sec. Butyl, tert.-Butyl, Pentyl, iso-Pentyl, Hexyl, Heptyl und Octyl. Zur Bezeichnung der Gruppen Methyl, Ethyl, Butyl oder auch tert.-Butyl werden auch die Abkürzungen Me, Et, Bu oder tBu verwendet.

Substituierte Alkylgruppen können, sofern nicht anders beschrieben (auch soweit sie Bestandteil anderer Reste sind), beispielsweise einen oder mehrere der nachfolgend genannten Substituenten tragen: Halogen, Hydroxy, Mercapto, $C_1$-$C_6$-Alkyloxy, Amino, Alkylamino, Dialkylamino, Cyano, Nitro, =O, -CHO, -COOH, -COO-$C_1$-$C_6$-Alkyl, -S-$C_1$-$C_6$-Alkyl.

Als Alkenylgruppen (auch soweit sie Bestandteil anderer Reste sind) werden verzweigte und unverzweigte Alkenylgruppen mit 2 bis 6 Kohlenstoffatomen, bevorzugt 2 bis 3 Kohlenstoffatomen genannt, soweit sie mindestens eine Doppelbindung aufweisen, beispielsweise auch oben genannte Alkylgruppen bezeichnet soweit sie mindestens eine Doppelbindung aufweisen, wie zum Beispiel Vinyl (soweit keine unbeständigen Enamine oder Enolether gebildet werden), Propenyl, iso-Propenyl, Butenyl, Pentenyl, Hexenyl.

Substituierte Alkenylgruppen können, sofern nicht anders beschrieben (auch soweit sie Bestandteil anderer Reste sind), beispielsweise einen oder mehrere der nachfolgend genannten Substituenten tragen: Halogen, Hydroxy, Mercapto, $C_1$-$C_6$-Alkyloxy, Amino, Alkylamino, Dialkylamino, Cyano, Nitro, =O, -CHO, -COOH, -COO-$C_1$-$C_6$-Alkyl, -S-$C_1$-$C_6$-Alkyl.

Als Alkinylgruppen (auch soweit sie Bestandteil anderer Reste sind) werden Alkinylgruppen mit 2 bis 10 Kohlenstoffatomen bezeichnet, soweit sie mindestens eine Dreifachbindung aufweisen, beispielsweise Ethinyl, Propargyl, Butinyl, Pentinyl, Hexinyl.

Substituierte Alkinylgruppen können, sofern nicht anders beschrieben (auch soweit sie Bestandteil anderer Reste sind), beispielsweise einen oder mehrere der nachfolgend genannten Substituenten tragen: Halogen, Hydroxy, Mercapto, $C_1$-$C_6$-Alkyloxy, Amino, Alkylamino, Dialkylamino, Cyano, Nitro, =O, -CHO, -COOH, -COO-$C_1$-$C_6$-Alkyl, -S-$C_1$-$C_6$-Alkyl.

Als Cycloalkylreste mit 3 - 6 Kohlenstoffatomen werden beispielsweise Cyclopropyl, Cyclobutyl, Cyclopentyl oder Cyclohexyl bezeichnet, die auch durch verzweigtes oder unverzweigtes Alkyl mit 1 bis 4 Kohlenstoffatomen, Hydroxy, und/oder Halogen oder wie zuvor definiert substituiert sein können. Als Halogen wird im allgemeinen Fluor, Chlor, Brom oder Jod bezeichnet.

Der Begriff Aryl steht für ein aromatisches Ringsystem mit 6 bis 10 Kohlenstoffatomen, das, soweit nicht anders beschrieben, beispielsweise einen oder mehrere der nachfolgend genannten Substituenten tragen kann: $C_1$-$C_6$-Alkyl, $C_1$-$C_6$-Alkyloxy, Halogen, Hydroxy, Mercapto, Amino, Alkylamino, Dialkylamino, $CF_3$, Cyano, Nitro, -CHO, -COOH, -COO-$C_1$-$C_6$-Alkyl, -S-$C_1$-$C_6$-Alkyl. Bevorzugter Arylrest ist Phenyl.

Als Beispiele für N-verknüpfte cyclische Reste der allgemeinen Formel $NR^5R^6$ seien genannt: Pyrrol, Pyrrolin, Pyrrolidin, 2-Methylpyrrolidin, 3-Methylpyrrolidin, Piperidin, Piperazin, N-Methylpiperazin, N-Ethylpiperazin, N-(n-Propyl)-piperazin, N-Benzylpiperazin, Morpholin, Thiomorpholin, Imidazol, Imidazolin, Imidazolidin, Pyrazol, Pyrazolin, Pyrazolidin, bevorzugt Morpholin, N-Benzylpiperazin, Piperazin, und Piperidin, wobei die genannten Heterocyclen durch Alkyl mit 1 bis 4 Kohlenstoffatomen, bevorzugt Methyl substituiert sein können.

Als C-verknüpfte 5- oder 6-gliedrige heterocyclische Ringe, die als Heteroatome Stickstoff, Sauerstoff oder Schwefel enthalten können, werden beispielsweise Furan, Tetrahydrofuran, 2-Methyltetrahydrofuran, 2-Hydroxyrnethylfuran, Tetrahydrofuranon, γ-Butylrolacton, α-Pyran, γ-Pyran, Dioxolan, Tetrahydropyran, Dioxan, Thiophen, Dihydrothiophen, Thiolan, Dithiolan, Pyrrol, Pyrrolin, Pyrrolidin, Pyrazol, Pyrazolin, Imidazol, Imidazolin, Imidazolidin, Triazol,Tetrazol, Pyridin, Piperidin, Pyridazin, Pyrimidin, Pyrazin, Piperazin, Triazin, Tetrazin, Morpholin, Thiomorpholin, Oxazol, Isoxazol, Oxazin, Thiazol, Isothiazol, Thiadiazol, Pyrazolidin genannt, wobei der Heterocyclus wie in den Definitionen angegeben, substituiert sein kann.

"=O" bedeutet ein über eine Doppelbindung verknüpftes Sauerstoffatom.

Die erfindungsgemäßen Verbindungen können beispielsweise gemäß der durch Schema 1 dargestellten Vorgehensweise synthetisiert werden.

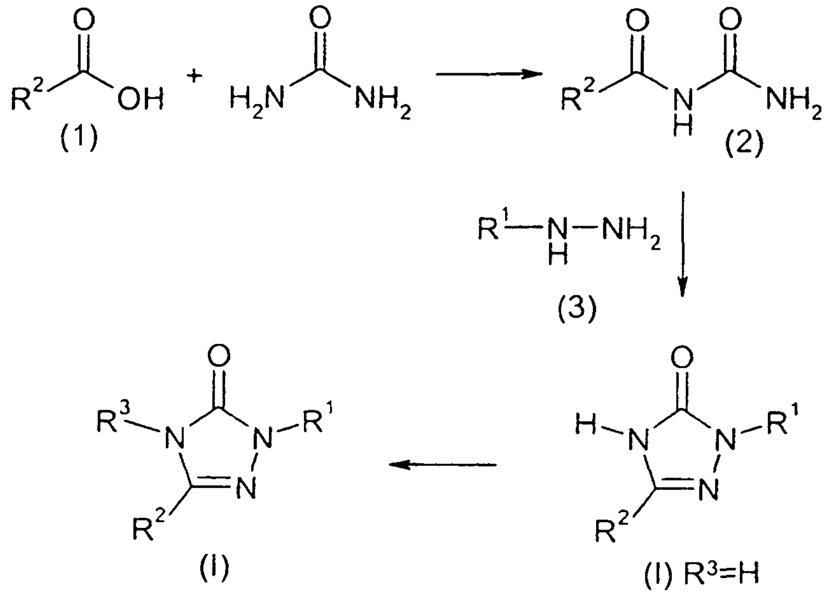

Schema 1:

Hierzu werden die Carbonsäurederivate (1) mit Harnstoff in die N-Acylharnstoffe (2) überführt. Aus diesen lassen sich durch Umsetzung mit entsprechend substituierten Hydrazin-Derivaten (3) die Triazolone der allgemeinen Formel (I) mit $R^3$=Wasserstoff erhalten, welche unter basischen Bedingungen über Alkylierung in die Verbindungen der allgemeinen Formel (I) transformiert werden können. Als Alkylierungsmittel kommen Chloride, Bromide, Jodide, Methansulfonate, Trifluormethansulfonate oder p-Toluolsulfonate in betracht.

Die vorliegende Erfindung soll anhand der folgenden allgemeinen Synthesevorschriften näher erläutert werden, ohne sie jedoch auf deren Gegenstand zu beschränken.

a) Allgemeine Arbeitsvorschrift zur Darstellung der N-Acyclharnstoffe (2):

0,1 Mol Carbonsäure-Derivat (1) werden zusammen mit 0,1 mol Harnstoff, 0.1 mol Triphenylphosphit und 0,1 mol Pyridin in 100 ml DMF gelöst und bis zum Ende der Reaktion bei 100°C gerührt (5- 24 Stunden). Das DMF wird im Vakuum verdampft, der Rückstand auf Wasser gegeben und aus Ethanol umkristallisiert. Ausbeute: 15 -50%.

b) Allgemeine Arbeitsvorschrift zur Darstellung der Triazolone (I, mit $R^3$=H):

0,01 Mol N-Acylharnstoff-Verbindung (2) werden mit 0,01 mol Hydrazin-Derivat (3) 1 - 6 Stunden in 30 ml Decalin bei 170°C gerührt. Man läßt abkühlen und filtriert die entstandenen Kristalle ab, wäscht mit Diethylether und kristallisiert aus Essigester um. Ausbeute: 20 - 70%.

c) Allgemeine Arbeitsvorschrift zur Darstellung der Triazolone (I, mit $R^3$=Methyl):

1,2 mmol Triazolon-Verbindung (I, mit $R^3$=H) werden mit 2,4 mmol Methyliodid und 2,4 mmol Kaliumcarbonat in 20 ml Aceton 3 Stunden bei 60°C gerührt. Das Aceton wird im Vakuum verdampft, der Rückstand in Wasser und Essigester aufgenommen und die wässrige Phase noch 2 mal mit Essigester extrahiert. Die vereinigten Essigester-Phasen werden über Natriumsulfat getrocknet und im Vakuum eingeengt. Die Reinigung erfolgt über Chromatographie an Kieselgel mit Methylenchlorid oder Toluol/Methanol mit anschließendem Urnkristatlieren aus Isopropanal/Benzin oder Ethanol. Ausbeute: 45 - 70%.

d) Allgemeine Arbeitsvorschrift zur Darstellung der Triazolone (I, mit $R^3$= 2-Dimethylaminoethyl):

1. 1,6 mmol Triazolon-Verbindung (I, mit $R^3$=H) werden in DMF gelöst, mit 1,6 mmol einer 60%igen Natriumhydrid-

Suspension in Öl 0,5 Stunden bei 80°C gerührt und anschließend mit 4,8 mmol Dibromethan versetzt. Es wird 4 Stunden bei 120°C gerührt. Das DMF wird im Vakuum verdampft, der Rückstand in Wasser und Essigester aufgenommen und die wässrige Phase noch 1 mal mit Essigester extrahiert. Die vereinigten Essigester-Phasen werden über Natriumsulfat getrocknet und im Vakuum eingeengt. Die Reinigung erfolgt über Chromatographie an Kieselgel mit Toluol/Essigester-Gemischen.

2. Die so gewonnene Menge an Bromethan-Derivat wird für 2 Stunden bei 1,8 bar mit einem Überschuß an Dimethylamin bei 100°C in Dioxan umgesetzt. Das Dioxan wird im Vakuum verdampft, der Rückstand in verd. wässriger Salzsäure-Lösung und Essigester aufgenommen. Die organsiche Phase wird noch 2 mal mit verd. wässriger Salzsäure-Lösung extrahiert und die vereinigten Extrakte mit Ammoniak-Lösung alkalisch gestellt. Die ammoniakalische Lösung wird 3 mal mit Essigester extrahiert und die vereinigten organischen Phasen über Natriumsulfat getrocknet und im Vakuum eingeengt. Der Rückstand wird in Ethanol aufgenommen, mit etherischer HCl versetzt und das Hydrochlorid mit wasserfreiem Ether ausgefällt. Ausbeute: 35 - 50%.

Gemäß den zuvor beschriebenen Verfahren oder in Anlehnung an Analogieverfahren können die in Tabelle 1 aufgeführten Verbindungen der allgemeinen Formel (I) hergestellt werden:

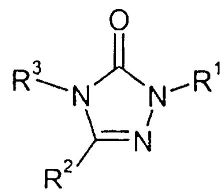

(I)

Tabelle 1:

| Beispiel | -$R^1$ | -$R^2$ | -$R^3$ | Schmp. [°C] | Chemische Bezeichnung |
|---|---|---|---|---|---|
| 1 | 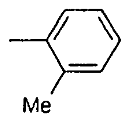 | 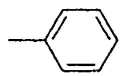 | -H | 208-211 | 2-(2-Methylphenyl)-5-phenyl-3H-1,2,4-triazol-3-on |
| 2 | 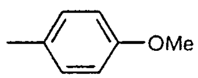 | 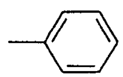 | -H | 220-224 | 2-(4-Methoxyphenyl)-5-phenyl-3H-1,2,4-triazol-3-on |
| 3 | 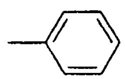 | 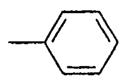 | 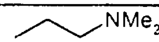 | 220-226 | 4-(2-N,N-Dimethylaminoethyl)-2,5-diphenyl-3H-1,2,4-triazol-3-on |
| 4 | 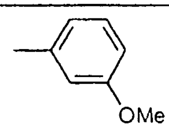 | 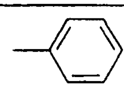 | -H | 224-226 | 2-(3-Methoxyphenyl)-5-phenyl-3H-1,2,4-triazol-3-on |
| 5 | 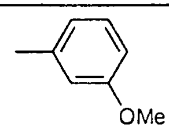 | 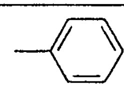 | -Methyl | 81-83 | 2-(3-Methoxyphenyl)-4-methyl-5-phenyl-3H-1,2,4-triazol-3-on |

| Beispiel | -$R^1$ | -$R^2$ | -$R^3$ | Schmp. [°C] | Chemische Bezeichnung |
|---|---|---|---|---|---|
| 6 | 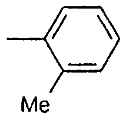 | 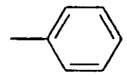 | -Methyl | 91-93 | 4-Methyl-2-(2-methylphenyl)-5-phenyl-3H-1,2,4-triazol-3-on |
| 7 | 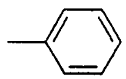 | 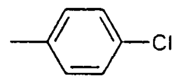 | -H | - | 5-(4-Chlorphenyl)-2-phenyl-3H-1,2,4-triazol-3-on |
| 8 | 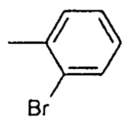 | 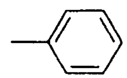 | -Methyl | 80-82 | 2-(2-Bromphenyl)-4-methyl-5-phenyl-3H-1,2,4-triazol-3-on |
| 9 | 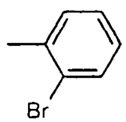 | 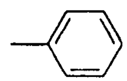 | 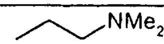 | 234-236 [a] | 2-(2-Bromphenyl)-4-(2-N,N-dimethylaminoethyl)-5-phenyl-3H-1,2,4-triazol-3-on |
| 10 | 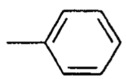 | 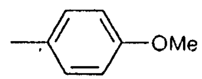 | -H | 233-235 | 5-(4-Methoxyphenyl)-2-phenyl-3H-1,2,4-triazol-3-on |
| 11 | 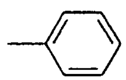 | 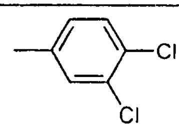 | -H | >300 | 5-(3,4-Dichlorphenyl)-2-phenyl-3H-1,2,4-triazol-3-on |
| 12 | 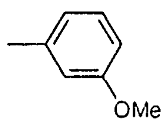 | 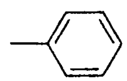 | 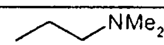 | 227-230 [a] | 4-(2-N,N-Dimethylaminoethyl)-2-(3-methoxyphenyl)-5-phenyl-3H-1,2,4-triazol-3-on |

| Beispiel | -$R^1$ | -$R^2$ | -$R^3$ | Schmp. [°C] | Chemische Bezeichnung |
|---|---|---|---|---|---|
| 13 | 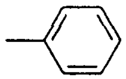 | 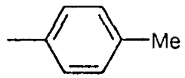 | -H | 255 | 5-(4-Methylphenyl)-2-phenyl-3H-1,2,4-triazol-3-on |
| 14 | 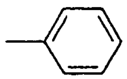 | 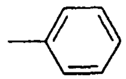 | 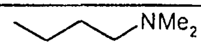 | 72 [a] | 4-(3-N,N-Dimethylaminopropyl)-2,5-diphenyl-3H-1,2,4-triazol-3-on |
| 15 | 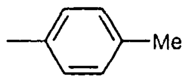 | 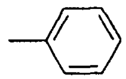 | -H | 234-236 | 2-(4-Methylphenyl)-5-phenyl-3H-1,2,4-triazol-3-on |
| 16 | 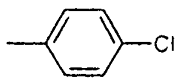 | 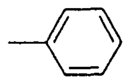 | -H | 258-268 | 2-(4-Chlorphenyl)-5-phenyl-3H-1,2,4-triazol-3-on |
| 17 | 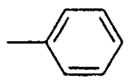 | 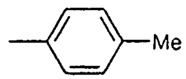 | -Methyl | 109-111 | 5-(4-Methylphenyl)-4-methyl-2-phenyl-3H-1,2,4-triazol-3-on |
| 18 | 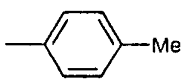 | 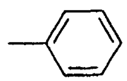 | -Methyl | 108 | 2-(4-Methylphenyl)-4-methyl-5-phenyl-3H-1,2,4-triazol-3-on |
| 19 | 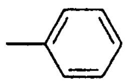 | 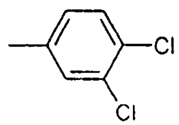 | -Methyl | 111-113 | 5-(3,4-Dichlorphenyl)-4-methyl-2-phenyl-3H-1,2,4-triazol-3-on |
| 20 | 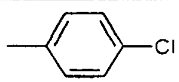 | 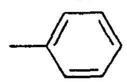 | -Methyl | 110-111 | 2-(4-Chlorphenyl)-4-methyl-2-phenyl-3H-1,2,4-triazol-3-on |
| 21 | 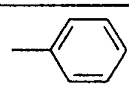 | 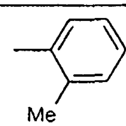 | -H | 176 | 5-(2-Methylphenyl)-2-phenyl-3H-1,2,4-triazol-3-on |

| Beispiel | -$R^1$ | -$R^2$ | -$R^3$ | Schmp. [°C] | Chemische Bezeichnung |
|---|---|---|---|---|---|
| 22 | 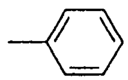 | 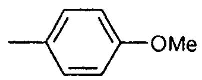 | -Methyl | 118 | 5-(4-Methoxyphenyl)-4-methyl-2-phenyl-3H-1,2,4-triazol-3-on |
| 23 | 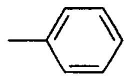 | 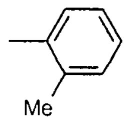 | -Methyl | 88-89 | 5-(2-Methylphenyl)-4-m,ethyl-2-phenyl-3H-1,2,4-triazol-3-on |
| 24 | 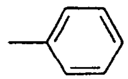 | 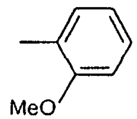 | -H | 217 | 5-(2-Methoxyphenyl)-2-phenyl-3H-1,2,4-triazol-3-on |
| 25 | 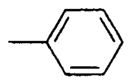 | 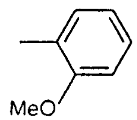 | -Methyl | 117 | 5-(2-Methoxyphenyl)-4-methyl-2-phenyl-3H-1,2,4-triazol-3-on |
| 26 | 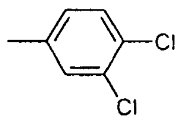 | 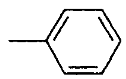 | -H | >280 | 2-(3,4-Dichlorphenyl)-5-phenyl-3H-1,2,4-triazol-3-on |
| 27 | 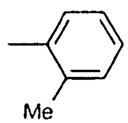 | 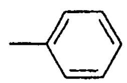 | 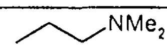 | 204-207 [a] | 4-(2-N,N-Dimethylaminoethyl)-2-(2-methylphenyl)-5-phenyl-3H-1,2,4-triazol-3-on |
| 28 | 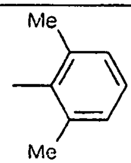 | 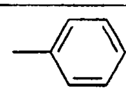 | -H | 253 | 2-(2,5-Dimethylphenyl)-5-phenyl-3H-1,2,4-triazol-3-on |

| Beispiel | -$R^1$ | -$R^2$ | -$R^3$ | Schmp. [°C] | Chemische Bezeichnung |
|---|---|---|---|---|---|
| 29 | 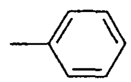 | 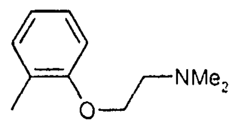 | -Methyl | 184-186 [a] | 4-Methyl-5-[2-(2-N,N-dimethylaminoethyl)phenyl]-2-phenyl-3H-1,2,4-triazol-3-on |
| 30 | 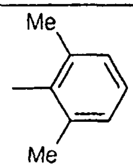 | 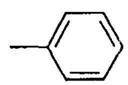 | -Methyl | 101-103 | 4-Methyl-2-(2,5-dimethylphenyl)-5-phenyl-3H-1,2,4-triazol-3-on |
| 31 | 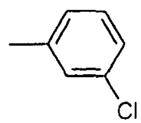 | 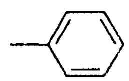 | -H | 264 | 2-(3-Chlorphenyl)-5-phenyl-3H-1,2,4-triazol-3-on |
| 32 | 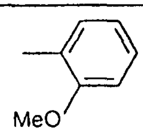 | 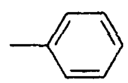 | -H | 275-277 | 2-(2-Methoxyphenyl)-5-phenyl-3H-1,2,4-triazol-3-on |
| 33 | 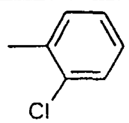 | 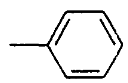 | -H | 257 | 2-(2-Chlorphenyl)-5-phenyl-3H-1,2,4-triazol-3-on |
| 34 | 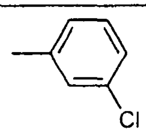 | 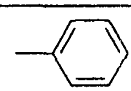 | -Methyl | 82-83 | 2-(3-Chlorphenyl)-4-methyl-5-phenyl-3H-1,2,4-triazol-3-on |

| Beispiel | -R¹ | -R² | -R³ | Schmp. [°C] | Chemische Bezeichnung |
|---|---|---|---|---|---|
| 35 | 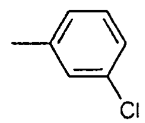 | 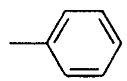 | 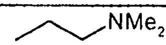 | 196 [a] | 2-(3-Chlorphenyl)-4-(2-N,N-dimethylaminoethyl)-5-phenyl-3H-1,2,4-triazol-3-on |
| 36 | 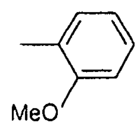 | 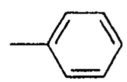 | -Methyl | 105 | 2-(2-Methoxyphenyl)-4-methyl-5-phenyl-3H-1,2,4-triazol-3-on |
| 37 | 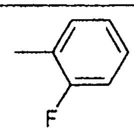 | 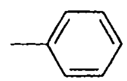 | -H | 238-240 | 2-(2-Fluorphenyl)-5-phenyl-3H-1,2,4-triazol-3-on |
| 38 | 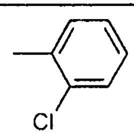 | 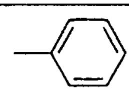 | 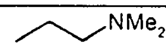 | 228-230 [a] | 2-(2-Chlorphenyl)-4-(2-N,N-dimethylaminoethyl)-5-phenyl-3H-1,2,4-triazol-3-on |
| 39 | 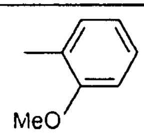 | 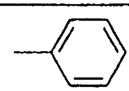 | 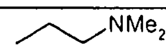 | 240-242 [a] | 2-(2-Methoxyphenyl)-4-(2-N,N-dimethylamino-ethyl)-5-phenyl-3H-1,2,4-triazol-3-on |
| 40 | 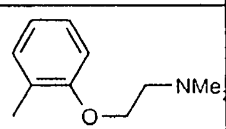 | 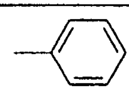 | -Methyl | 188-191 [a] | 4-Methyl-2-[2-(2-N,N-dimethylaminoethyl)phenyl]-5-phenyl-3H-1,2,4-triazol-3-on |

| Beispiel | $-R^1$ | $-R^2$ | $-R^3$ | Schmp. [°C] | Chemische Bezeichnung |
|---|---|---|---|---|---|
| 41 | 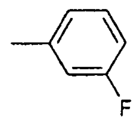 | 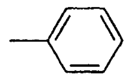 | -H | 253-254 | 2-(3-Fluorphenyl)-5-phenyl-3H-1,2,4-triazol-3-on |
| 42 | 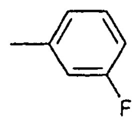 | 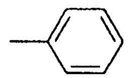 | -Methyl | 81 | 2-(3-Fluorphenyl)-4-methyl-5-phenyl-3H-1,2,4-triazol-3-on |
| 43 | 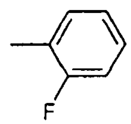 | 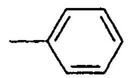 | 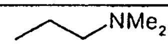 | 201-202 [a] | 2-(2-Fluorphenyl)-4-(2-N,N-dimethylaminoethyl)-5-phenyl-3H-1,2,4-triazol-3-on |
| 44 | 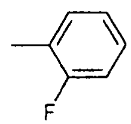 | 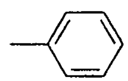 | -Methyl | 65-66 | 2-(2-Fluorphenyl)-4-methyl-5-phenyl-3H-1,2,4-triazol-3-on |
| 45 | 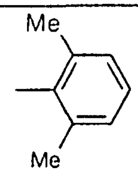 | 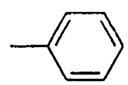 | 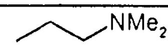 | 215-217 [a] | 4-(2-N,N-Dimethylaminoethyl)-2-(2,5-dimethylphenyl)-5-phenyl-3H-1,2,4-triazol-3-on |
| 46 | 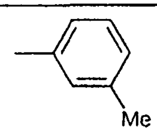 | 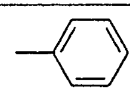 | -H | 207-208 [a] | 2-(3-Methylphenyl)-5-phenyl-3H-1,2,4-triazol-3-on |

| Beispiel | -$R^1$ | -$R^2$ | -$R^3$ | Schmp. [°C] | Chemische Bezeichnung |
|---|---|---|---|---|---|
| 47 | 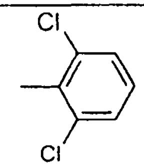 | 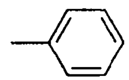 | -H | 297 | 2-(2,5-Dichlorphenyl)-5-phenyl-3H-1,2,4-triazol-3-on |
| 48 | 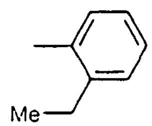 | 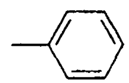 | -H | 209-210 | 2-(2-Ethylphenyl)-5-phenyl-3H-1,2,4-triazol-3-on |
| 49 | 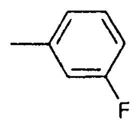 | 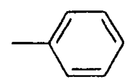 | 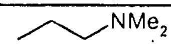 | 201-203 [a] | 2-(3-Fluorphenyl)-4-(2-N,N-dimethylaminoethyl)-5-phenyl-3H-1,2,4-triazol-3-on |
| 50 | 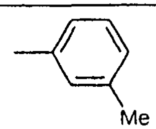 | 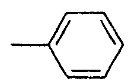 | -Methyl | 91-92 | 4-Methyl-2-(3-methylphenyl)-5-phenyl-3H-1,2,4-triazol-3-on |
| 51 | 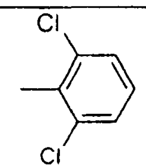 | 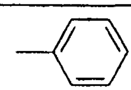 | -Methyl | 156-158 | 2-(2,5-Dichlorphenyl)-4-methyl-5-phenyl-3H-1,2,4-triazol-3-on |
| 52 | 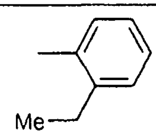 | 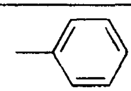 | -Methyl | 72-74 | 2-(2-Ethylphenyl)-4-methyl-5-phenyl-3H-1,2,4-triazol-3-on |

| Beispiel | $-R^1$ | $-R^2$ | $-R^3$ | Schmp. [°C] | Chemische Bezeichnung |
|---|---|---|---|---|---|
| 53 | 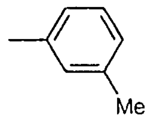 | 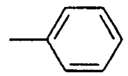 | 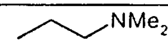 | 204-206 [a] | 4-(2-N,N-Dimethylaminoethyl)-2-(3-methylphenyl)-5-phenyl-3H-1,2,4-triazol-3-on |
| 54 | 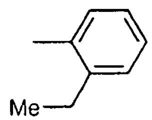 | 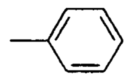 | 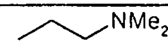 | 203 [a] | 2-(2-Ethylphenyl)-4-(2-N,N-dimethylaminoethyl)-5-phenyl-3H-1,2,4-triazol-3-on |
| 55 | 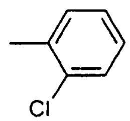 | 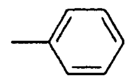 | -Methyl | 94-96 | 2-(2-Chlorphenyl)-4-methyl-5-phenyl-3H-1,2,4-triazol-3-on |
| 56 | 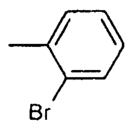 | 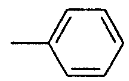 | -H | 246-264 | 2-(2-Bromphenyl)-5-phenyl-3H-1,2,4-triazol-3-on |
| 57 | 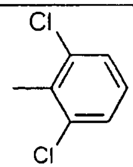 | 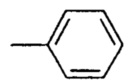 | 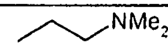 | 222-224 [a] | 2-(2,5-Dichlorphenyl)-4-(2-N,N-dimethylamino-ethyl)-5-phenyl-3H-1,2,4-triazol-3-on |
| 58 | 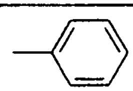 | 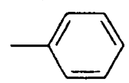 | -Methyl | | 4-Methyl-2,5-diphenyl-3H-1,2,4-triazol-3-on |
| 59 | 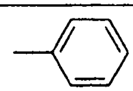 | 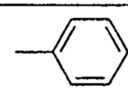 | -H | 234-237 | 2,5-Diphenyl-3H-1,2,4-triazol-3-on |

| Beispiel | -R[1] | -R[2] | -R[3] | Schmp. [°C] | Chemische Bezeichnung |
|---|---|---|---|---|---|
| 60 | 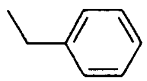 | 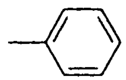 | -H | 220 | 2-Benzyl-5-phenyl-3H-1,2,4-triazol-3-on |
| 61 | 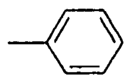 | -Methyl | -H | 185-187 | 5-Methyl-2-phenyl-3H-1,2,4-triazol-3-on |
| 62 | 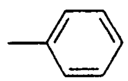 | -Methyl | -Methyl | 96-98 | 4,5-Dimethyl-2-phenyl-3H-1,2,4-triazol-3-on |
| 63 * | -Methyl | 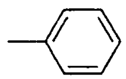 | -H | 217-218 | 2-Methyl-5-phenyl-3H-1,2,4-triazol-3-on |
| 64 | 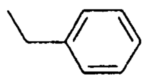 | 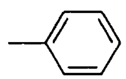 | -Methyl | 62-64 | 2-Benzyl-4-methyl-5-phenyl-3H-1,2,4-triazol-3-on |
| 65 * | -tert.-Butyl | 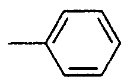 | -H | 171-173 | 2-tert.-Butyl-5-phenyl-3H-1,2,4-triazol-3-on |
| 66 | 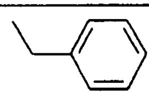 | 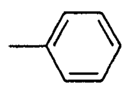 | 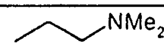 | 188-190 [a] | 2-Benzyl-4-(2-N,N-dimethylaminoethyl)-5-phenyl-3H-1,2,4-triazol-3-on |
| 67 * | -Methyl | 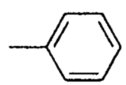 | -Methyl | 140-142 | 2,4-Dimethyl-5-phenyl-3H-1,2,4-triazol-3-on |
| 68 * | -Methyl | 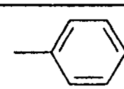 | 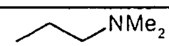 | 192-194 [a] | 2-Methyl-4-(2-N,N-dimethylaminoethyl)-5-phenyl-3H-1,2,4-triazol-3-on |

| Beispiel | $-R^1$ | $-R^2$ | $-R^3$ | Schmp. [°C] | Chemische Bezeichnung |
|---|---|---|---|---|---|
| 69 * | -tert.-Butyl | 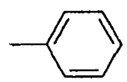 | -Methyl | 67-68 | 2-tert.-Butyl-4-methyl-5-phenyl-3H-1,2,4-triazol-3-on |
| 70 | 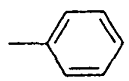 | -Methyl | 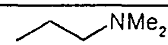 | 139 [a] | 5-Methyl-4-(2-N,N-dimethylaminoethyl)-2-phenyl-3H-1,2,4-triazol-3-on |
| 71 * | -tert.-Butyl | 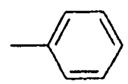 | 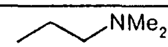 | 162 [a] | 2-tert.-Butyl-4-(2-N,N-dimethylaminoethyl)-5-phenyl-3H-1,2,4-triazol-3-on |
| 72 | 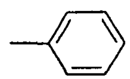 | 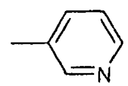 | 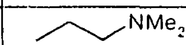 | 229 [b] | 4-(2-N,N-Dimethylaminoethyl)-2-phenyl-5-(3-pyridyl)-3H-1,2,4-triazol-3-on |
| 73 | 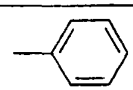 | 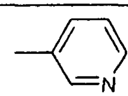 | -H | 246 [a] | 2-Phenyl-5-(3-pyridyl)-3H-1,2,4-triazol-3-on |
| 74 | 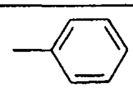 | 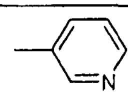 | -Methyl | 185-187 [a] | 4-Methyl-2-phenyl-5-(3-pyridyl)-3H-1,2,4-triazol-3-on |
| 75 | 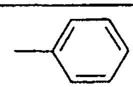 | 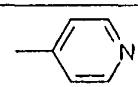 | -H | 290 | 2-Phenyl-5-(4-pyridyl)-3H-1,2,4-triazol-3-on |

a) xHCl; b) x 2HCl; *) Referenz - Verbindungen

Der Nachweis der Wirkung am AMPA Rezeptor wurde mittels Elektrophysiologie an neuronalen Zellen (Patch-Clamp-Methode) geführt (M. L. Mayer, L. Vyklicky and G. L. Westbrook, J. Physiol. 415 (1989) 329-350). Die Testung erfolgte bei einer Testkonzentration von 100 µM.

Tabelle 2: Hemmung des Kainat-induzierten Signals am AMPA-Rezeptor

| Beispiel | AMPA Inh. [%] |
|---|---|
| 1 | 58 |
| 5 | 58 |
| 8 | 87 |
| 22 | 50 |
| 25 | 72 |
| 27 | 64 |
| 32 | 73 |
| 33 | 97 |

| Beispiel | AMPA Inh. [%] |
|---|---|
| 37 | 86 |
| 42 | 96 |
| 44 | 76 |
| 46 | 54 |
| 48 | 63 |
| 50 | 71 |
| 55 | 80 |
| 56 | 96 |

Der Nachweis der Affinität zur "$Na^+$ Kanal site 2"-Bindungsstelle wurde wie von G.B. Brown (J. Neurosci. 6 (1986) 2064) beschrieben geführt. Die Testung erfolgte typischerweise bei einer Testkonzentration von 10µM.

Tabelle 3:

| Beispiel | Ki [µm] |
|---|---|
| 27 | 5,41 |
| 35 | 2.08 |
| 49 | 6.58 |
| 53 | 5.78 |

Die oben beschriebenen Ergebnisse zeigen, daß die Triazolonderivate der allgemeinen Formel (I) bei neurodegenerativen Erkrankungen sowie Gehirnischämie verschiedener Genese eingesetzt werden können. Hierunter fallen beispielsweise: Status epileptikus, Hypoglykämie, Hypoxie, Anoxie, Gehirntrauma, Gehirnoedem, amyotrope laterale Sklerose, Huntington's Disease, Morbus Alzheimer, Hypotonie, Herzinfarkt, Hirndruck (erhöhter intrakranialer Druck), ischämischer und hämorrhagischer Stroke, globale cerebrale Ischämie bei Herzstillstand, Diabetische Polyneuropathie, Tinitus, perinatale Asphyxie, Psychose, Schizophrenie, Depression und Morbus Parkinson.

Die Verbindungen der allgemeinen Formel (I) können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, insbesondere Lösungen zur Injektion (s.c., i.v., i.m.) und Infusion, Säfte, Emulsionen oder dispersible Pulver. Hierbei soll der Anteil der pharmazeutisch wirksamen Verbindung(en) jeweils im Bereich von 0,1 bis 90 Gew.-%, bevorzugt 0,5 bis 50 Gew.-% der Gesamtzusammensetzung liegen, d.h. in Mengen die ausreichend sind, um den unten angegebenen Dosierungsbereich zu erreichen.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder

Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z. B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzukker) Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise parenteral - insbesondere auf dem Wege der Infusion - intravenös. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Die Dosierung für die intravenöse Anwendung liegt bei 1 - 1000 mg pro Stunde, vorzugsweise zwischen 5 - 500 mg pro Stunde.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über der Tag zu verteilen.

Ferner können die Verbindungen der allgemeinen Formel (I) bzw. deren Säureadditionssalze auch mit andersartigen Wirkstoffen kombiniert werden.

Die nachfolgenden Beispiele illustrieren die vorliegende Erfindung ohne sie jedoch in ihrem Umfang zu beschränken:

<u>Pharmazeutische Formulierungsbeispiele</u>

| A) | Tabletten | pro Tablette |
|---|---|---|
| | Wirkstoff | 100 mg |

(fortgesetzt)

| A) | Tabletten | pro Tablette |
|---|---|---|
| | Milchzucker<br>Maisstärke<br>Polyvinylpyrrolidon<br>Magnesiumstearat | 140 mg<br>240 mg<br>15 mg<br>5 mg<br>500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet , knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff<br>Milchzucker<br>Maisstärke<br>Mikrokristalline Cellulose<br>Polyvinylpyrrolidon<br>Natrium-carboxymethylstärke<br>Magnesiumstearat | 80 mg<br>55 mg<br>190 mg<br>35 mg<br>15 mg<br>23 mg<br>2 mg<br>400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | Wirkstoff<br>Natriumchlorid<br>Aqua pro inj. | 50 mg<br>50 mg<br>5 ml |

Der Wirkstoff wird bei Eigen-pH oder gegebenenfalls bei pH 5,5 bis 6,5 in Wasser gelöst und mit Natriumchlorid als Isotonans versetzt. die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

**1.** Verbindungen der allgemeinen Formel (I) zur Verwendung als Arzneimittel,

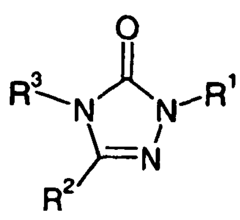

(I)

worin

$R^1$ $C_6$-$C_{10}$-Aryl, das gegebenenfalls direkt oder über eine Alkylenbrücke mit 1 bis 4 C-Atomen durch einen oder mehrere der Reste Halogen, Nitro, $-CF_3$, -CN, $-OR^4$, $-COOR^4$, $-OCOR^4$, $-SR^5$, $-SO_2R^5$, $-OSO_2R^5$, $-NR^6A^7$, $C_1$-$C_4$-Alkyl, $C_2$-$C_4$-Alkenyl oder $C_2$-$C_4$-Alkinyl substituiert sein kann;

$R^2$ $C_6$-$C_{10}$-Aryl, das gegebenenfalls direkt oder über eine Alkylenbrücke mit 1 bis 4 C-Atomen durch einen oder mehrere der Reste Halogen, Nitro, $-CF_3$, -CN, $-OR^4$, $-COOR^4$, $-OCOR^4$, $-SR^5$, $-SO_2R^5$, $-OSO_2R^5$, $-NR^6R^7$, $C_1$-$C_4$-Alkyl, $C_2$-$C_4$-Alkenyl oder $C_2$-$C_4$-Alkinyl substituiert sein kann;

$R^2$ ein $C_1$-$C_6$-Alkyl-, $C_2$-$C_6$-Alkenyl- oder $C_2$-$C_6$-Alkinyl-Rest, der gegebenenfalls durch Phenyl, $-NA^6A^7$, Halogen, Nitro, $CF_3$, CN oder $-OA^4$ substituiert sein kann;

$R^2$ ein C-verknüpfter 5- oder 6-gliedriger gesättigter oder ungesättigter Heterocyclus, der als Heteroatome 1, 2, 3 oder 4 Atome ausgewählt aus der Gruppe Sauerstoff, Stickstoff oder Schwefel enthalten kann und der gegebenenfalls durch $C_1$-$C_6$-Alkyl oder Benzyl substituiert sein kann;

$R^3$ Wasserstoff oder ein $C_1$-$C_6$-Alkyl-, $C_2$-$C_6$-Alkenyl- oder $C_2$-$C_6$-Alkinyl-Rest, der gegebenenfalls durch $-NR^6R^7$, Halogen, Nitro, $CF_3$, CN oder $-OR^4$ substituiert sein kann;

$R^4$ Wasserstoff, gegebenenfalls durch Halogen oder $-NR^6R^7$ substituiertes $C_1$-$C_4$-Alkyl oder ein Phenyl- oder Benzyl-Rest, der gegebenenfalls eine oder mehrere Methoxygruppen tragen kann;

$R^5$ Wasserstoff, $C_1$-$C_4$-Alkyl, Phenyl oder Benzyl, wobei der Phenyl oder Benzyrest gegebenenfalls ein- oder mehrfach durch Methoxy substituiert sein können;

$R^6$ Wasserstoff, $C_3$-$C_6$-Cycloalkyl, $C_1$-$C_6$-Alkyl, $C_2$-$C_6$-Alkenyl oder $C_2$-$C_6$-Alkinyl, die jeweils ein- oder mehrfach durch Phenyl, Benzyl oder $-OR^4$ substituiert sein können;

$R^6$ $C_6$-$C_{10}$-Aryl, das gegebenenfalls durch Halogen, $OR^4$, $C_1$-$C_4$-Alkyl, $-SO_3H$, oder $-COOR^4$ substituiert sein kann;

$R^7$ Wasserstoff, $C_3$-$C_6$-Cycloalkyl, $C_1$-$C_6$-Alkyl, $C_2$-$C_6$-Alkenyl oder $C_2$-$C_6$-Alkinyl, die jeweils ein- oder mehrfach durch Phenyl, Benzyl oder $-OR^4$ substituiert sein können;

$R^7$ $C_6$-$C_{10}$-Aryl, das gegebenenfalls durch Halogen, $OR^4$, $C_1$-$C_4$-Alkyl, $-SO_3H$, oder $-COOR^4$ substituiert sein kann,
oder

$R^6$ und $R^7$ bilden zusammen mit dem Stickstoffatom einen gesättigten oder ungesättigten 5- oder 6-gliedrigen Ring, der als weitere Heteroatome Stickstoff, Sauerstoff oder Schwefel enthalten kann, wobei der Heterocyclus durch eine verzweigte oder unverzweigte Alkylgruppe mit 1 bis 4 Kohlenstoffatomen, durch Phenyl oder durch Benzyl substituiert sein kann, bedeuten,

gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**2.** Verbindungen der allgemeinen Formel (I) gemäß Anspruch 1 zur Verwendung als Arzneimittel, worin

$R^1$ Phenyl, das gegebenenfalls direkt oder über eine Alkylenbrücke mit 1 bis 4 C-Atomen durch einen oder mehrere der Reste Fluor, Chlor, Brom, Nitro, $-CF_3$, -CN, $-OR^4$, $-COOR^4$, $-OCOR^4$, $-NR^6R^7$, $C_1$-$C_4$-Alkyl, $C_2$-$C_4$-Alkenyl oder $C_2$-$C_4$-Alkinyl substituiert sein kann;

$R^2$ Phenyl, das gegebenenfalls direkt oder über eine Alkylenbrücke mit 1 bis 4 C-Atomen durch einen oder mehrere der Reste Fluor, Chlor, Brom, Nitro, $-CF_3$, -CN, $-OR^4$, $-COOR^4$, $-OCOR^4$, $-NR^6R^7$, $C_1$-$C_4$-Alkyl, $C_2$-$C_4$-Alkenyl oder $C_2$-$C_4$-Alkinyl substituiert sein kann;

$R^2$ $C_1$-$C_4$-Alkyl, das gegebenenfalls durch Phenyl substituiert sein kann;

$R^2$ ein C-verknüpfter 5- oder 6-gliedriger gesättigter oder ungesättigter Heterocyclus, der als Heteroatome 1, 2, 3 oder 4 Atome ausgewählt aus der Gruppe Sauerstoff oder Stickstoff enthalten kann und der gegebenenfalls durch $C_1$-$C_4$-Alkyl oder Benzyl substituiert sein kann;

$R^3$ Wasserstoff oder ein $C_1$-$C_4$-Alkyl-Rest, der gegebenenfalls durch $-NR^6R^7$, Fluor, Chlor, Brom, Nitro, $CF_3$, CN oder $-OR^4$ substituiert sein kann;

$R^4$ Wasserstoff, gegebenenfalls durch Halogen oder $-NR^6R^7$ substituiertes $C_1$-$C_4$-Alkyl oder ein Phenyl- oder Benzyl-Rest, der gegebenenfalls eine oder mehrere Methoxygruppen tragen kann;

$R^6$ Wasserstoff, $C_1$-$C_4$-Alkyl, $C_2$-$C_4$-Alkenyl oder $C_2$-$C_4$-Alkinyl, die jeweils ein- oder mehrfach durch Phenyl, Benzyl oder $-OR^4$ substituiert sein können,

$R^6$ Phenyl oder Benzyl, die gegebenenfalls durch Halogen, $OR^4$, $C_1$-$C_4$-Alkyl, $-SO_3H$, oder $-COOR^4$ substituiert sein können;

$R^7$ Wasserstoff, $C_1$-$C_4$-Alkyl, $C_2$-$C_4$-Alkenyl oder $C_2$-$C_4$-Alkinyl, die jeweils ein- oder mehrfach durch Phenyl, Benzyl oder $-OR^4$ substituiert sein können,

$R^7$ Phenyl oder Benzyl, die gegebenenfalls durch Halogen, $OR^4$, $C_1$-$C_4$-Alkyl, $-SO_3H$, oder $-COOR^4$ substituiert sein können;
oder

$R^6$ und $R^7$ bilden zusammen mit dem Stickstoffatom einen gesättigten oder ungesättigten 5- oder 6-gliedrigen Ring, der als weitere Heteroatome Stickstoff oder Sauerstoff enthalten kann, wobei der Heterocyclus durch eine verzweigte oder unverzweigte Alkylgruppe mit 1 bis 4 Kohlenstoffatomen, durch Phenyl oder durch Benzyl substituiert sein kann, bedeuten,

gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**3.** Verbindungen der allgemeinen Formel (I) gemäß Anspruch 1 oder 2 zur Verwendung als Arzneimittel, worin

$R^1$ Phenyl, das gegebenenfalls durch einen oder mehrere der Reste Fluor, Chlor, Brom, $-OR^4$ oder $C_1$-$C_4$-Alkyl substituiert sein kann;

$R^2$ $C_1$-$C_4$-Alkyl oder Phenyl, das gegebenenfalls durch einen oder mehrere der Reste Fluor, Chlor, Brom, $-OR^4$ oder $C_1$-$C_4$-Alkyl substituiert sein kann;

$R^2$ ein C-verknüpfter 5- oder 6-gliedriger Heterocyclus ausgewählt aus der Gruppe Furan, Pyran, Pyrrol, Pyrazol, Imidazol, Triazol,Tetrazol, Pyridin, Pyridazin, Pyrimidin, Pyrazin, Triazin, Oxazol oder Isoxazol;

$R^3$ Wasserstoff oder ein $C_1$-$C_4$-Alkyl-Rest, der gegebenenfalls durch $-NR^6R^7$, Chlor, Brom oder -OH substituiert sein kann;

$R^4$ Wasserstoff, gegebenenfalls durch Chlor, Brom oder $-NR^6R^7$ substituiertes $C_1$-$C_4$-Alkyl oder ein Phenyl- oder Benzyl-Rest;

$R^6$ Wasserstoff, $C_1$-$C_4$-Alkyl, Phenyl oder Benzyl;

$R^7$ Wasserstoff, $C_1$-$C_4$-Alkyl, Phenyl oder Benzyl;
oder

$R^6$ und $R^7$ bilden zusammen mit dem Stickstoffatom einen gesättigten oder ungesättigten 5- oder 6-gliedrigen und gegebenenfalls durch $C_1$-$C_4$-Alkyl oder Benzyl substituierten Ring, ausgewählt aus der Gruppe Piperidin, Piperazin, Morpholin, Pyrrol oder Pyrrolidin, bedeuten, gegebenenfalls in Form ihrer Racemate, ihrer Enantiomere, in Form ihrer Diastereomere und ihrer Gemische, sowie gegebenenfalls

ihrer pharmakologisch unbedenklichen Säureadditionssalze.

**4.** Verbindungen der allgemeinen Formel (I) gemäß einem der Ansprüche 1-3 zur Verwendung als Arzneimittel, worin

$R^1$ Phenyl, das gegebenenfalls durch einen oder mehrere der Reste Fluor, Chlor, Brom, Methyl, Ethyl, Propyl oder -$OR^4$ substituiert sein kann;

$R^2$ Methyl, Ethyl, Propyl, Butyl oder Phenyl, das gegebenenfalls durch einen oder mehrere der Reste Fluor, Chlor, Brom, Methyl, Ethyl, Propyl oder -$OR^4$ substituiert sein kann;

$R^2$ ein C-verknüpfter Heterocyclus ausgewählt aus der Pyrrol, Pyrazol, Imidazol, Triazol, Pyridin, Pyridazin, Pyrimidin, Pyrazin oder Triazin;

$R^3$ Wasserstoff, Methyl, Ethyl oder Propyl die gegebenenfalls durch -$NR^6R^7$, Chlor, Brom oder -OH substituiert sein können;

$R^4$ Wasserstoff, Methyl, Ethyl oder Propyl, die gegebenenfalls durch -$NR^6R^7$, Chlor, Brom oder -OH substituiert sein können;

$R^6$ Wasserstoff, Methyl Ethyl, Propyl oder Benzyl;

$R^7$ Wasserstoff, Methyl Ethyl, Propyl oder Benzyl;
oder

$R^6$ und $R^7$ bilden zusammen mit dem Stickstoffatom einen gegebenenfalls durch Methyl Ethyl, Propyl oder Benzyl substituierten Ring, ausgewählt aus der Gruppe Piperidin, Piperazin, Morpholin, Pyrrol oder Pyrrolidin, bedeuten,

gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**5.** Verbindungen der allgemeinen Formel (I) gemäß einem der Ansprüche 1-4 zur Verwendung als Arzneimittel, worin

$R^1$ Phenyl, das gegebenenfalls durch einen oder mehrere der Reste Fluor, Chlor, Brom, Methyl, Ethyl, Propyl oder -$OR^4$ substituiert sein kann;

$R^2$ Methyl, Ethyl, Propyl, Butyl oder Phenyl, das gegebenenfalls durch einen oder mehrere der Reste Fluor, Chlor, Brom, Methyl, Ethyl, Propyl oder -$QR^4$ substituiert sein kann;

$R^2$ Pyrrol, Pyrazol, Imidazol, Pyridin, Pyridazin, Pyrimidin oder Pyrazin;

$R^3$ Wasserstoff, Methyl, Ethyl oder Propyl, die gegebenenfalls durch -$NR^6R^7$ sein können;

$R^4$ Methyl, Ethyl, Propyl, -$CH_2CH_2$-$NR^6R^7$ oder -$CH_2CH_2CH_2$-$NR^6R^7$;

$R^6$ Wasserstoff, Methyl, Ethyl, Propyl oder Benzyl;

$R^7$ Wasserstoff, Methyl, Ethyl, Propyl oder Benzyl;
bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**6.** Verbindungen der allgemeinen Formel (I) gemäß einem der Ansprüche 1-5 zur Verwendung als Arzneimittel, worin

$R^1$ Phenyl, das gegebenenfalls durch einen oder mehrere der Reste Fluor, Chlor, Brom, Methyl, Ethyl oder -$OR^4$ substituiert sein kann;

$R^2$ Methyl, Phenyl, das gegebenenfalls durch einen oder mehrere der Reste Chlor, Methyl oder -$OR^4$ substituiert sein kann;

$R^3$ Wasserstoff, Methyl, $-CH_2CH_2-NR^6R^7$ oder $-CH_2CH_2CH_2-NR^6R^7$;

$R^4$ Methyl oder $-CH_2CH_2-NR^6R^7$;

$R^6$ Methyl;

$R^7$ Methyl, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**7.** Verwendung einer Verbindung der allgemeinen Formel (I) gemäß Anspruch 1 bis 6 zur Herstellung eines Arzneimittels zur Behandlung von neurodegenerativen Erkrankungen sowie Gehirnischämie verschiedener Genese.

**8.** Pharmazeutische Zubereitungen, enthaltend als Wirkstoff eine oder mehrere Verbindungen der allgemeinen Formel (I) gemäß den Ansprüchen 1 bis 6 oder deren physiologisch verträgliche Säureadditionssalze in Kombination mit üblichen Hilfs- und/oder Trägerstoffen.

**Claims**

**1.** Compounds of general formula (I) for use as pharmaceutical compositions,

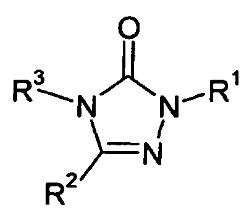

(I)

wherein

$R^1$ denotes $C_6$-$_{10}$-aryl, which may optionally be substituted directly or via a $C_{1-4}$-alkylene bridge by one or more of the groups halogen, nitro, $-CF_3$, -CN, $-OR^4$, $-COOR^4$, $-OCOR^4$, $-SR^5$, $-SO_2R^5$, $-OSO_2R^5$, $-NR^6R^7$, $C_1$-$C_4$-alkyl, $C_2$-$C_4$-alkenyl or $C_2$-$C_4$-alkynyl;

$R^2$ denotes $C_6$-$C_{10}$-aryl, which may optionally be substituted directly or via a $C_{1-4}$-alkylene bridge by one or more of the groups halogen, nitro, $-CF_3$, -CN, $-OR^4$, $-COOR^4$, $-OCOR^4$, $-SR^5$, $-SO_2R^5$, $-OSO_2R^5$, $-NR^6R^7$, $C_1$-$C_4$-alkyl, $C_2$-$C_4$-alkenyl or $C_{2}$-$_{4}$-alkynyl;

$R^2$ denotes a $C_1$-$_6$-alkyl, $C_2$-$_6$-alkenyl or $C_2$-$_6$-alkynyl group, which may optionally be substituted by phenyl, $-NR^6R^7$, halogen, nitro, $CF_3$, CN or $-OR^4$;

$R^2$ denotes a C-linked 5- or 6-membered saturated or unsaturated heterocycle, which may contain as heteroatoms 1, 2, 3 or 4 atoms selected from the group comprising oxygen, nitrogen or sulphur and which may optionally be substituted by $C_1$-$C_6$-alkyl or benzyl;

$R^3$ denotes hydrogen or a $C_1$-$C_6$-alkyl, $C_2$-$C_6$-alkenyl or $C_2$-$C_6$-alkynyl group, which may optionally be substituted by $-NR^6R^7$, halogen, nitro, $CF_3$, CN or $-OR^4$;

$R^4$ denotes hydrogen, $C_1$-$_4$-alkyl optionally substituted by halogen or $-NR^6R^7$ or a phenyl or benzyl group, which may optionally carry one or more methoxy groups;

$R^5$ denotes hydrogen, $C_1$-$_4$-alkyl, phenyl or benzyl, wherein the phenyl or benzyl group may optionally be mono- or polysubstituted by methoxy;

$R^6$ denotes hydrogen, $C_3$-$_6$-cycloalkyl, $C_1$-$_6$-alkyl, $C_2$-$_6$-alkenyl or $C_2$-$C_6$-alkynyl, each of which may be mono- or polysubstituted by phenyl, benzyl or -$OR^4$;

$R^6$ denotes $C_6$-$_{10}$-aryl, which may optionally be substituted by halogen, $OR^4$, $C_1$-$C_4$-alkyl, -$SO_3H$ or -$COOR^4$;

$R^7$ denotes hydrogen, $C_3$-$_6$-cycloalkyl, $C_1$-$_6$-alkyl, $C_2$-$_6$-alkenyl or $C_2$-$_6$-alkynyl, each of which may be mono- or polysubstituted by phenyl, benzyl or -$OR^4$;

$R^7$ denotes $C_6$-$_{10}$-aryl, which may optionally be substituted by halogen, $OR^4$, $C_1$-$C_4$-alkyl, -$SO_3H$, or -$COOR^4$;
or

$R^6$ and $R^7$ together with the nitrogen atom form a saturated or unsaturated 5- or 6-membered ring which may contain nitrogen, oxygen or sulphur as further heteroatoms, whilst the heterocycle may be substituted by a branched or unbranched alkyl group having 1 to 4 carbon atoms, or by phenyl or benzyl,
optionally in the form of their racemates, their enantiomers, in the form of their diastereomers and mixtures thereof, and
optionally the pharmacologically acceptable acid addition salts thereof.

**2.** Compounds of general formula (I) according to claim 1 for use as pharmaceutical compositions, wherein

$R^1$ denotes phenyl, which may optionally be substituted directly or via a $C_{1-4}$-alkylene bridge by one or more of the groups fluorine, chlorine, bromine, nitro, -$CF_3$, -CN, -$OR^4$, -$COOR^4$, -$OCOR^4$, -$NR^6R^7$, $C_{1-4}$-alkyl, $C_{2-4}$-alkenyl or $C_{2-4}$-alkynyl;

$R^2$ denotes phenyl, which may optionally be substituted directly or via a $C_{1-4}$-alkylene bridge by one or more of the groups fluorine, chlorine, bromine, nitro, -$CF_3$, -CN, -$OR^4$, -$COOR^4$, -$OCOR^4$, -$NR^6R^7$, $C_{1-4}$-alkyl, $C_{2-4}$-alkenyl or $C_{2-4}$-alkynyl;

$R^2$ denotes $C_1$-$C_4$-alkyl, which may optionally be substituted by phenyl;

$R^2$ denotes a C-linked 5- or 6-membered saturated or unsaturated heterocycle, which may contain as heteroatoms 1, 2, 3 or 4 atoms selected from the group comprising oxygen or nitrogen and which may optionally be substituted by $C_1$-$C_4$-alkyl or benzyl;

$R^3$ denotes hydrogen or a $C_{1-4}$-alkyl group, which may optionally be substituted by -$NR^6R^7$, fluorine, chlorine, bromine, nitro, $CF_3$, CN or -$OR^4$;

$R^4$ denotes hydrogen, $C_1$-$C_4$-alkyl optionally substituted by halogen or -$NR^6R^7$ or a phenyl or benzyl group, which may optionally carry one or more methoxy groups;

$R^6$ denotes hydrogen, $C_1$-$_4$-alkyl, $C_2$-$_4$-alkenyl or $C_2$-$_4$-alkynyl, each of which may be mono- or polysubstituted by phenyl, benzyl or -$OR^4$,

$R^6$ denotes phenyl or benzyl, which may optionally be substituted by halogen, $OR^4$, $C_{1-4}$-alkyl, -$SO_3H$ or -$COOR^4$;

$R^7$ denotes hydrogen, $C_{1-4}$-alkyl, $C_{2-4}$-alkenyl or $C_{2-4}$-alkynyl, each of which may be mono- or polysubstituted by phenyl, benzyl or -$OR^4$,

$R^7$ denotes phenyl or benzyl, which may optionally be substituted by halogen, $OR^4$, $C_{1-4}$-alkyl, -$SO_3H$ or -$COOR^4$;
or

$R^6$ and $R^7$ together with the nitrogen atom form a saturated or unsaturated 5- or 6-membered ring, which may contain nitrogen or oxygen as further heteroatoms, whilst the heterocycle may be substituted by a branched or unbranched alkyl group having 1 to 4 carbon atoms, or by phenyl or benzyl,

optionally in the form of their racemates, their enantiomers, in the form of their diastereomers and mixtures thereof, and optionally the pharmacologically acceptable acid addition salts thereof.

**3.** Compounds of general formula (I) according to claim 1 or 2 for use as pharmaceutical compositions, wherein

$R^1$ denotes phenyl which may optionally be substituted by one or more of the groups fluorine, chlorine, bromine, $-OR^4$ or $C_{1-4}$-alkyl;

$R^2$ denotes $C_{1-4}$-alkyl or phenyl which may optionally be substituted by one or more of the groups fluorine, chlorine, bromine, $-OR^4$ or $C_{1-4}$-alkyl;

$R^2$ denotes a C-linked 5- or 6-membered heterocycle selected from the group comprising furan, pyran, pyrrole, pyrazole, imidazole, triazole, tetrazole, pyridine, pyridazine, pyrimidine, pyrazine, triazine, oxazole or isoxazole;

$R^3$ denotes hydrogen or a $C_1$-$C_4$-alkyl group, which may optionally be substituted by $-NR^6R^7$, chlorine, bromine or -OH;

$R^4$ denotes hydrogen, $C_{1-4}$-alkyl optionally substituted by chlorine, bromine or $-NR^6R^7$ or a phenyl or benzyl group;

$R^6$ denotes hydrogen, $C_{1-4}$-alkyl, phenyl or benzyl;

$R^7$ denotes hydrogen, $C_{1-4}$-alkyl, phenyl or benzyl;
or

$R^6$ and $R^7$ together with the nitrogen atom form a saturated or unsaturated 5- or 6-membered ring optionally substituted by $C_{1-4}$-alkyl or benzyl, selected from the group comprising piperidine, piperazine, morpholine, pyrrole or pyrrolidine,
optionally in the form of their racemates, their enantiomers, in the form of their diastereomers and mixtures thereof, and optionally the pharmacologically acceptable acid addition salts thereof.

**4.** Compounds of general formula (I) according to one of claims 1 to 3 for use as pharmaceutical compositions, wherein

$R^1$ denotes phenyl, which may optionally be substituted by one or more of the groups fluorine, chlorine, bromine, methyl, ethyl, propyl or $-OR^4$;

$R^2$ denotes methyl, ethyl, propyl, butyl or phenyl, which may optionally be substituted by one or more of the groups fluorine, chlorine, bromine, methyl, ethyl, propyl or $-OR^4$;

$R^2$ denotes a C-linked heterocycle selected from pyrrole, pyrazole, imidazole, triazole, pyridine, pyridazine, pyrimidine, pyrazine or triazine;

$R^3$ denotes hydrogen, methyl, ethyl or propyl which may optionally be substituted by $-NR^6R^7$, chlorine, bromine or -OH;

$R^4$ denotes hydrogen, methyl, ethyl or propyl, which may optionally be substituted by $-NR^6R^7$, chlorine, bromine or -OH;

$R^6$ denotes hydrogen, methyl ethyl, propyl or benzyl;

$R^7$ denotes hydrogen, methyl ethyl, propyl or benzyl;
or

$R^6$ and $R^7$ together with the nitrogen atom form a ring optionally substituted by methyl, ethyl, propyl or benzyl, selected from the group comprising piperidine, piperazine, morpholine, pyrrole or pyrrolidine,
optionally in the form of their racemates, their enantiomers, in the form of their diastereomers and mixtures thereof, and optionally the pharmacologically acceptable acid addition salts thereof.

**5.** Compounds of general formula (I) according to one of claims 1 to 4 for use as pharmaceutical compositions, wherein

$R^1$ denotes phenyl, which may optionally be substituted by one or more of the groups fluorine, chlorine, bromine, methyl, ethyl, propyl or $-OR^4$;

$R^2$ denotes methyl, ethyl, propyl, butyl or phenyl, which may optionally be substituted by one or more of the groups fluorine, chlorine, bromine, methyl, ethyl, propyl or $-OR^4$;

$R^2$ denotes pyrrole, pyrazole, imidazole, pyridine, pyridazine, pyrimidine or pyrazine;

$R^3$ denotes hydrogen, methyl, ethyl or propyl, which may optionally be substituted by $-NR^6R^7$;

$R^4$ denotes methyl, ethyl, propyl, $-CH_2CH_2-NR^6R^7$ or $-CH_2CH_2CH_2-NR^6R^7$;

$R^6$ denotes hydrogen, methyl, ethyl, propyl or benzyl;

$R^7$ denotes hydrogen, methyl, ethyl, propyl or benzyl;
optionally in the form of their racemates, their enantiomers, in the form of their diastereomers and mixtures thereof, and optionally the pharmacologically acceptable acid addition salts thereof.

**6.** Compounds of general formula (I) according to one of claims 1 to 5 for use as pharmaceutical compositions, wherein

$R^1$ denotes phenyl, which may optionally be substituted by one or more of the groups fluorine, chlorine, bromine, methyl, ethyl or $-OR^4$;

$R^2$ denotes methyl, phenyl, which may optionally be substituted by one or more of the groups chlorine, methyl or $-OR^4$;

$R^3$ denotes hydrogen, methyl, $-CH_2CH_2-NR^6R^7$ or $-CH_2CH_2CH_2-NR^6R^7$;

$R^4$ denotes methyl or $-CH_2CH_2-NR^6R^7$;

$R^6$ denotes methyl;

$R^7$ denotes methyl,
optionally in the form of their racemates, their enantiomers, in the form of their diastereomers and mixtures thereof, and optionally the pharmacologically acceptable acid addition salts thereof.

**7.** Use of a compound of general formula (I) according to claims 1 to 6 for preparing a pharmaceutical composition for treating neurodegenerative disorders as well as cerebral ischaemia of various origins.

**8.** Pharmaceutical preparations containing as active substance one or more compounds of general formula (I) according to claims 1 to 6 or the physiologically acceptable acid addition salts thereof in conjunction with conventional excipients and/or carriers.

**Revendications**

**1.** Composés de formule générale (I) destinés à être utilisés comme médicament

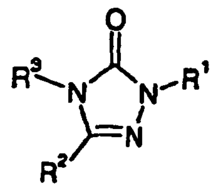

(I)

où

$R^1$ représente $C_6$-$C_{10}$-aryle, qui peut éventuellement être substitué directement ou par le biais d'un pont alkylène ayant 1 à 4 atomes de carbone par un ou plusieurs des groupements halogène, nitro, $-CF_3$, -CN, $-OR^4$, $-COOR^4$, $-OCOR^4$, $-SR^5$, $-SO_2R^5$, $-OSO_2R^5$, $-NR^6R^7$, $C_1$-$C_4$-alkyle, $C_2$-$C_4$-alcényle ou $C_2$-$C_4$-alcynyle ;

$R^2$ représente $C_6$-$C_{10}$-aryle, qui peut éventuellement être substitué directement ou par le biais d'un pont alkylène ayant 1 à 4 atomes de carbone par un ou plusieurs des groupements halogène, nitro, $-CF_3$, -CN, $-OR^4$, $-COOR^4$, $-OCOR^4$, $-SR^5$, $-SO_2R^5$, $-OSO_2R^5$, $-NR^6R^7$, $C_1$-$C_4$-alkyle, $C_2$-$C_4$-alcényle ou $C_2$-$C_4$-alcynyle ;

$R^2$ représente un groupement $C_1$-$C_6$-alkyle, $C_2$-$C_6$-alcényle ou $C_2$-$C_6$-alcynyle, qui peut éventuellement être substitué par phényle, $-NR^6R^7$, halogène, nitro, $CF_3$, CN ou $-OR^4$ ;

$R^2$ représente un hétérocycle saturé ou insaturé à 5 ou 6 chaînons lié par C, qui peut contenir comme hétéroatomes 1, 2, 3 ou 4 atomes choisis dans le groupe oxygène, azote ou soufre et qui peut éventuellement être substitué par $C_1$-$C_6$-alkyle ou benzyle ;

$R^3$ représente l'hydrogène ou un groupement $C_1$-$C_6$-alkyle, $C_2$-$C_6$-alcényle ou $C_2$-$C_6$-alcynyle, qui peut éventuellement être substitué par $-NR^6R^7$, halogène, nitro, $CF_3$, CN ou $-OR^4$ ;

$R^4$ représente l'hydrogène, un groupement $C_1$-$C_4$-alkyle éventuellement substitué par halogène ou $-NR^6R^7$ ou un groupement phényle ou benzyle, qui peut éventuellement porter un ou plusieurs groupes méthoxy ;

$R^5$ représente l'hydrogène, $C_1$-$C_4$-alkyle, phényle ou benzyle, où le groupement phényle ou benzyle peut éventuellement être substitué une ou plusieurs fois par méthoxy ;

$R^6$ représente l'hydrogène, $C_3$-$C_6$-cycloalkyle, $C_1$-$C_6$-alkyle, $C_2$-$C_6$-alcényle ou $C_2$-$C_6$-alcynyle, qui peuvent être substitués dans chaque cas une ou plusieurs fois par phényle, benzyle ou $-OR^4$ ;

$R^6$ représente $C_6$-$C_{10}$-aryle, qui peut éventuellement être substitué par halogène, $-OR^4$, $C_1$-$C_4$-alkyle, $-SO_3H$ ou $-COOR^4$ ;

$R^7$ représente l'hydrogène, $C_3$-$C_6$-cycloalkyle, $C_1$-$C_6$-alkyle, $C_2$-$C_6$-alcényle ou $C_2$-$C_6$-alcynyle, qui peuvent être substitués dans chaque cas une ou plusieurs fois par phényle, benzyle ou $-OR^4$;

$R^7$ représente $C_6$-$C_{10}$-aryle, qui peut éventuellement être substitué par halogène, $-OR^4$, $C_1$-$C_4$-alkyle, $-SO_3H$ ou $-COOR^4$ ;

ou

$R^6$ et $R^7$ forment avec l'atome d'azote un cycle à 5 ou 6 chaînons saturé ou insaturé, qui peut contenir comme autres hétéroatomes l'azote, l'oxygène ou le soufre, où l'hétérocycle peut être substitué par un groupe alkyle ramifié ou non ramifié ayant 1 à 4 atomes de carbone, par phényle ou par benzyle,

éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

**2.** Composés de formule générale (I) selon la revendication 1 destinés à être utilisés comme médicament où

$R^1$ représente phényle, qui peut éventuellement être substitué directement ou par le biais d'un pont alkylène ayant 1 à 4 atomes de carbone par un ou plusieurs des groupements fluor, chlore, brome, nitro, $-CF_3$, -CN, $-OR^4$, $-COOR^4$, $-OCOR^4$, $-NR^6R^7$, $C_1$-$C_4$-alkyle, $C_2$-$C_4$-alcényle ou $C_2$-$C_4$-alcynyle ;

$R^2$ représente phényle, qui peut éventuellement être substitué directement ou par le biais d'un pont alkylène ayant 1 à 4 atomes de carbone par un ou plusieurs des groupements fluor, chlore, brome, nitro, $-CF_3$, -CN, $-OR^4$, $-COOR^4$, $-OCOR^4$, $-NR^6R^7$, $C_1$-$C_4$-alkyle, $C_2$-$C_4$-alcényle ou $C_2$-$C_4$-alcynyle ;

$R^2$ représente $C_1$-$C_4$-alkyle qui peut éventuellement être substitué par phényle ;

$R^2$ représente un hétérocycle saturé ou insaturé à 5 ou 6 chaînons lié par C, qui peut contenir comme hétéroatomes 1, 2, 3 ou 4 atomes choisis dans le groupe oxygène ou azote et qui peut éventuellement être substitué par $C_1$-$C_4$-alkyle ou benzyle;

$R^3$ représente l'hydrogène ou un groupement $C_1$-$C_4$-alkyle, qui peut éventuellement être substitué par $-NR^6R^7$, le fluor, le chlore, le brome, nitro, $CF_3$, CN ou $-OR^4$ ;

$R^4$ représente l'hydrogène, $C_1$-$C_4$-alkyle éventuellement substitué par halogène ou $-NR^6R^7$ ou un groupement phényle ou benzyle, qui peut éventuellement porter un ou plusieurs groupes méthoxy ;

$R^6$ représente l'hydrogène, $C_1$-$C_4$-alkyle, $C_2$-$C_4$-alcényle ou $C_2$-$C_4$-alcynyle, qui peuvent être substitués dans chaque cas une ou plusieurs fois par phényle, benzyle ou -$OR^4$ ;
$R^6$ représente phényle ou benzyle, qui peuvent éventuellement être substitués par halogène, -$OR^4$, $C_1$-$C_4$-alkyle, -$SO_3H$ ou -$COOR^4$ ;
$R^7$ représente l'hydrogène, $C_1$-$C_4$-alkyle, $C_2$-$C_4$-alcényle ou $C_2$-$C_4$-alcynyle, qui peuvent être substitués dans chaque cas une ou plusieurs fois par phényle, benzyle ou -$OR^4$ ;
$R^7$ représente phényle ou benzyle, qui peuvent éventuellement être substitués par halogène, -$OR^4$, $C_1$-$C_4$-alkyle, -$SO_3H$ ou -$COOR^4$;
ou
$R^6$ et $R^7$ forment avec l'atome d'azote un cycle à 5 ou 6 chaînons saturé ou insaturé, qui peut contenir comme autres hétéroatomes l'azote ou l'oxygène, où l'hétérocycle peut être substitué par un groupe alkyle ramifié ou non ramifié ayant 1 à 4 atomes de carbone, par phényle ou par benzyle,
éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

**3.** Composés de formule générale (I) selon la revendication 1 ou 2 destinés à être utilisés comme médicament où
$R^1$ représente phényle, qui peut éventuellement être substitué par un ou plusieurs des groupements fluor, chlore, brome, -$OR^4$ ou $C_1$-$C_4$-alkyle ;
$R^2$ représente $C_1$-$C_4$-alkyle ou phényle, qui peut éventuellement être substitué par un ou plusieurs des groupements fluor, chlore, brome, -$OR^4$ ou $C_1$-$C_4$-alkyle ;
$R^2$ représente un hétérocycle à 5 ou 6 chaînons lié par C choisi dans le groupe furane, pyrane, pyrrole, pyrazole, imidazole, triazole, tétrazole, pyridine, pyridazine, pyrimidine, pyrazine, triazine, oxazole ou isoxazole ;
$R^3$ représente l'hydrogène ou un groupement $C_1$-$C_4$-alkyle, qui peut éventuellement être substitué par -$NR^6R^7$, le chlore, le brome ou -OH ;
$R^4$ représente l'hydrogène, $C_1$-$C_4$-alkyle éventuellement substitué par le chlore, le brome ou -$NR^6R^7$ ou un groupement phényle ou benzyle ;
$R^6$ représente l'hydrogène, $C_1$-$C_4$-alkyle, phényle ou benzyle ;
$R^7$ représente l'hydrogène, $C_1$-$C_4$-alkyle, phényle ou benzyle;
ou
$R^6$ et $R^7$ forment avec l'atome d'azote un cycle à 5 ou 6 chaînons saturé ou insaturé et éventuellement substitué par $C_1$-$C_4$-alkyle ou benzyle, choisi dans le groupe pipéridine, pipérazine, morpholine, pyrrole ou pyrrolidine, éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

**4.** Composés de formule générale (I) selon l'une des revendications 1-3 destinés à être utilisés comme médicament où
$R^1$ représente phényle, qui peut éventuellement être substitué par un ou plusieurs des groupements fluor, chlore, brome, méthyle, éthyle, propyle ou -$OR^4$ ;
$R^2$ représente méthyle, éthyle, propyle, butyle ou phényle, qui peut éventuellement être substitué par un ou plusieurs des groupements fluor, chlore, brome, méthyle, éthyle, propyle ou -$OR^4$ ;
$R^2$ représente un hétérocycle lié par C choisi parmi pyrrole, pyrazole, imidazole, triazole, pyridine, pyridazine, pyrimidine, pyrazine ou triazine ;
$R^3$ représente l'hydrogène, méthyle, éthyle ou propyle, qui peuvent éventuellement être substitués par -$NR^6R^7$, le chlore, le brome ou -OH ;
$R^4$ représente l'hydrogène, méthyle, éthyle ou propyle, qui peuvent éventuellement être substitués par-$NR^6R^7$, le chlore, le brome ou -OH ;
$R^6$ représente l'hydrogène, méthyle, éthyle, propyle ou benzyle ;
$R^7$ représente l'hydrogène, méthyle, éthyle, propyle ou benzyle ;
ou
$R^6$ et $R^7$ forment avec l'atome d'azote un cycle éventuellement substitué par méthyle, éthyle, propyle ou benzyle, choisi dans le groupe pipéridine, pipérazine, morpholine, pyrrole ou pyrrolidine, éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

**5.** Composés de formule générale (I) selon l'une des revendications 1-4 destinés à être utilisés comme médicament où
$R^1$ représente phényle, qui peut éventuellement être substitué par un ou plusieurs des groupements fluor, chlore, brome, méthyle, éthyle, propyle ou -$OR^4$ ;

$R^2$ représente méthyle, éthyle, propyle, butyle ou phényle, qui peut éventuellement être substitué par un ou plusieurs des groupements fluor, chlore, brome, méthyle, éthyle, propyle ou $-OR^4$ ;
$R^2$ représente pyrrole, pyrazole, imidazole, pyridine, pyridazine, pyrimidine ou pyrazine ;
$R^3$ représente l'hydrogène, méthyle, éthyle ou propyle, qui peuvent éventuellement être substitués par $-NR^6R^7$ ;
$R^4$ représente méthyle, éthyle, propyle, $-CH_2CH_2-NR^6R^7$ ou $-CH_2CH_2CH_2-NR^6R^7$ ;
$R^6$ représente l'hydrogène, méthyle, éthyle, propyle ou benzyle ;
$R^7$ représente l'hydrogène, méthyle, éthyle, propyle ou benzyle ;
éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

**6.** Composés de formule générale (I) selon l'une des revendications 1-5 destinés à être utilisés comme médicament où
$R^1$ représente phényle, qui peut éventuellement être substitué par un ou plusieurs des groupements fluor, chlore, brome, méthyle, éthyle ou $-OR^4$ ;
$R^2$ représente méthyle, phényle, qui peut éventuellement être substitué par un ou plusieurs des groupements chlore, méthyle ou $-OR^4$ ;
$R^3$ représente l'hydrogène, méthyle, $-CH_2CH_2-NR^6R^7$ ou $-CH_2CH_2CH_2-NR^6R^7$ ;
$R^4$ représente méthyle ou $-CH_2CH_2-NR^6R^7$ ;
$R^6$ représente méthyle ;
$R^7$ représente méthyle, éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

**7.** Utilisation d'un composé de formule générale (I) selon les revendications 1 à 6 pour la production d'un médicament pour le traitement des maladies neurodégénératives ainsi que de l'ischémie cérébrale de genèse diverse.

**8.** Préparations pharmaceutiques contenant comme principe actif un ou plusieurs composés de formule générale (I) selon les revendications 1 à 6 ou leurs sels d'addition d'acide physiologiquement acceptables en combinaison avec des adjuvants et/ou supports habituels.